# EUROPEAN PATENT SPECIFICATION

(11) **EP 4 317 451 B1**
(45) Date of publication and mention of the grant of the patent: **09.09.2026**
(21) Application number: 22781293.0
(22) Date of filing: 31.03.2022
(51) Int. Cl.: C12Q 1/00, G01N 1/34, G01N 33/48, C07K 1/14, G01N 33/53

(54) **METHOD FOR TREATING BIOLOGICAL SAMPLE**
VERFAHREN ZUR BEHANDLUNG EINER BIOLOGISCHEN PROBE
PROCÉDÉ DE TRAITEMENT D'UN ÉCHANTILLON BIOLOGIQUE

(30) Priority: 01.04.2021 JP 2021062584
(43) Date of publication of application: 07.02.2024
(73) Proprietor: Fujirebio Inc., Tokyo 107-0052 (JP)
(72) Inventor: TERAO, Azusa, Tokyo 163-0410 (JP); AOYAGI, Katsumi, Tokyo 163-0410 (JP)
(74) Representative: Hoffmann Eitle
(86) International application number: PCT/JP2022/016827
(87) International publication number: WO 2022/211084

(56) References cited:
- WO-A1-2011/081075
- WO-A1-2019/194280
- WO-A1-2021/015123
- WO-A2-2008/035991
- JP-A- 2005 134 372
- JP-A- 2006 189 358
- JP-A- 2008 541 115
- JP-A- 2012 511 145
- JP-A- 2014 209 919
- JP-A- 2017 072 603
- JP-A- 2019 152 666
- JP-A- 2021 012 197
- JP-A- H 085 637
- JP-A- H04 233 459
- JP-A- H05 223 820
- JP-A- S5 991 900
- JP-B2- 3 140 057
- US-A1- 2005 037 386
- US-A1- 2006 199 203

## Description

### Field

The present invention relates to a method for treating a biological sample, and the like.

### Background

For purification or analysis of a target molecule (for example, a protein), development of a technique capable of efficiently extracting the target molecule from a biological sample is required. When a target molecule is extracted from a biological sample, classically, a method for physically destroying a tissue using a device such as a homogenizer or a sonicator and extracting the target molecule is widely used. However, such a method is complicated because the device needs to be washed for each sample, and is not suitable for treating many samples.

A formalin fixed paraffin embedded (FFPE) tissue sample is a clinical material that can be easily preserved for a long period of time while a form thereof is highly maintained. An FFPE tissue sample has been collected for various organs and diseases for a long period of time, and therefore has attracted attention in recent years as an analysis target of a target molecule. However, due to use of formalin and paraffin in an FFPE tissue sample (for example, crosslinking by formalin or paraffin contamination), it is difficult to efficiently extract a target molecule (for example, a protein) from the FFPE tissue sample and to analyze the extracted target molecule with high reliability.

Regarding a sample treating method for extracting a target molecule from a biological sample such as a tissue sample, for example, the following method have been reported.

Patent Literature 1 discloses a method for producing a tissue extract, the method including treating a tissue sample with a cell dispersing enzyme and a membrane solubilizing surfactant.

Patent Literature 2 discloses a method for extracting a target molecule from a tissue sample using a specific device.

Patent Literatures 3 and 4 disclose a method for extracting a protein from a biological sample, the method including a two-stage temperature treatment step of heating a solution containing a biological sample and a surfactant at a temperature of 95°C or higher and then incubating the solution at a temperature higher than 60°C.

### Citation List

### Patent Literature

Patent Literature 1: JP 2004-313019 A
Patent Literature 2: WO 2005/116256 A
Patent Literature 3: WO 2006/122898 A
Patent Literature 4: WO 2010/063837 A

### Summary

### Technical Problem

An object of the present invention is to provide a method for treating a biological sample such as a formalin fixed paraffin embedded (FFPE) tissue sample, the method being simple and suitable for treating many samples.

### Solution to Problem

As a result of intensive studies, the present inventors have found that a simple treatment method for heating a mixed liquid that contains a biological sample containing a target molecule and contains an ionic surfactant, and then mixing the heated mixed liquid with a specific ionic surfactant is useful for analysis of the target molecule in the biological sample, and have completed the present invention.

### Effects of Invention

According to the present invention utilizing a combination of a first ionic surfactant and a second ionic surfactant, and heating, a target molecule can be efficiently extracted from a biological sample, and a treated sample useful for analysis of the target molecule in the biological sample can be obtained.

In addition, according to the present invention, release of fixation (for example, cleavage of methylene crosslinking with formalin) in a fixed tissue sample can be promoted, the target molecule can be efficiently extracted, and a treated sample useful for analysis of the target molecule can be obtained.
paraffin in a paraffin embedded tissue sample can be removed from a solution without requiring replacement of a container, the target molecule can be efficiently extracted, and the extracted target molecule can be easily analyzed with high reliability by any method.

### Brief Description of Drawings

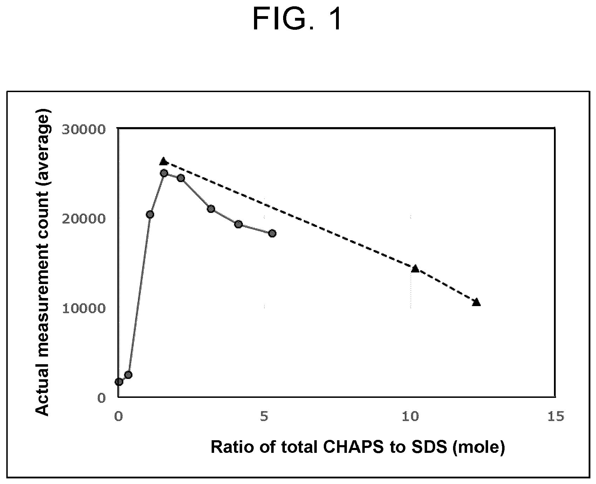

FIG. 1 is a diagram illustrating a relationship between a signal count value and a molar ratio of a zwitterionic surfactant (3-[(3-cholamidopropyl) dimethylammonio]-1-propanesulfonate (CHAPS)) during a treatment with a second ionic surfactant to an anionic surfactant (SDS) during a treatment with a first ionic surfactant.

### Description of Embodiments

### 1. Method for treating biological sample

The present invention provides a method for treating a biological sample, the method including the following (1) and (2):
(1) heating a solution that contains a biological sample containing a target molecule and contains a first ionic surfactant; and
(2) mixing the heated solution with a second ionic surfactant containing an ionic moiety having a charge opposite to that of an ionic moiety in the first ionic surfactant.

### (1) Heating of solution

In the method of the present invention, a solution that contains a biological sample containing a target molecule and contains a first ionic surfactant is heated.

The target molecule is any molecule contained in the biological sample. The target molecule includes not only an endogenous natural molecule but also an exogenous synthetic molecule. This is because the biological sample (for example, an individual multicellular organism itself or a part thereof, or a unicellular organism) may contain a synthetic molecule due to a circumstance such as absorption of a compound (for example, administration of a medicament, ingestion of a food component, or application to the skin) or exposure to a compound (for example, exposure to an endocrine disruptor in an environment or a treatment of a cultured cell with a compound). Examples of the target molecule include a protein (including an oligopeptide, a polypeptide, and a glycoprotein), a polysaccharide, a lipid, and a low molecular weight compound. The target molecule is preferably a protein. Examples of the protein include a secreted factor (for example, an immunoglobulin such as an autoantibody), a growth factor, an enzyme, a receptor (for example, a cell membrane receptor or a channel), a ligand, an adaptor, a cell adhesion factor, an extracellular matrix, a transporter, a cytoplasmic protein, a nuclear protein (for example, a transcription factor), and a disordered protein (for example, a β-amyloid aggregate).

The target molecule may be a marker molecule for a condition such as a specific disease. A suitable example of such a marker molecule is a tumor marker molecule. Examples of the tumor marker molecule include a carcinoembryonic antigen (CEA), α-fetoprotein (AFP), PIVKA-II (protein induced by vitamin K absence or antagonist), a carbohydrate antigen 19-9 (cancer antigen 19-9: CA19-9), a carbohydrate antigen 125 (CA125), a carbohydrate antigen 15-3 (CA15-3), a prostate-specific antigen (PSA), a cytokeratin 19 fragment (cyfra), a squamous cell carcinoma-associated antigen (SCC), a soluble mesothelin-associated protein, p53, and thyroglobulin. As another suitable example of the marker molecule, a gastric mucosal atrophy marker molecule (for example, pepsinogen (I and II)), a liver fibrosis marker molecule (for example, type IV collagen), an interstitial pneumonia marker molecule (for example, a sialylated carbohydrate antigen KL-6) may be used. The target molecule may also be a viral antigen. Examples of the viral antigen include a hepatitis virus antigen (for example, a hepatitis B virus surface (HBs) antigen or a hepatitis C virus (HCV) core protein).

The biological sample used in the present invention is a sample derived from an organism and contains a target molecule. The target molecule in the biological sample may be a molecule included in a biological boundary structure (for example, a lipid bilayer membrane such as a cell membrane or a nuclear membrane, or an outer coat such as a protein coat) (for example, a molecule included in a cytoplasm or a nucleus) or may be a molecule included in a biological boundary structure (for example, a molecule embedded in a lipid bilayer membrane or an outer coat). The first ionic surfactant and heating can act synergistically on the biological sample to efficiently destroy an interaction between substances constituting a biological boundary structure (for example, a lipid-lipid interaction, a lipid-protein interaction, or a protein-protein interaction). Examples of such a biological sample include a sample containing an individual multicellular organism itself or a part thereof, a unicellular organism, or a virus.

Examples of the multicellular organism include an animal such as a mammal (for example, a primate such as a human or a monkey; a rodent such as a mouse, a rat, or a rabbit; or an ungulate such as a cow, a pig, a goat, a horse, or a sheep) or a bird (for example, a chicken), a plant, fish, an insect, and a parasite. When an individual multicellular organism itself is used as the biological sample, the multicellular organism may be a multicellular organism other than a human. The multicellular organism may also be in a condition such as a specific disease (for example, a cancer, a neurodegenerative disease, or an inflammatory disease).

Examples of the part of the multicellular organism include a tissue collected from the multicellular organism or a part thereof (for example, a tissue section), a body fluid collected from the multicellular organism, and a cell derived from the multicellular organism (for example, a primary cultured cell or a cell line) or a culture thereof. Examples of such a tissue include an animal tissue such as a respiratory tissue (for example, lung, trachea, bronchus, pharynx, nasal cavity, or nasal sinus), a digestive tissue (for example, stomach, small intestine, large intestine, colon, or rectum), pancreas, kidney, liver, thymus, spleen, heart, thyroid, adrenal gland, prostate, ovary, uterus, brain, skin, or bone marrow, and a plant tissue such as a leaf, a stem, a fruit, or a rhizome. Examples of such a body fluid include blood, urine, saliva, lymph, a tissue fluid, a cerebrospinal fluid, ascites, sweat, semen, tear, mucus, milk, a pleural fluid, a bronchoalveolar lavage fluid, and an amniotic fluid. This is because such a body fluid may contain an entity (for example, a cell or a virus) including a biological boundary structures as described above.

Examples of the unicellular organism include a microorganism (for example, a prokaryote or a eukaryote).

Examples of the virus include a virus having an outer coat structure such as a protein coat.

According to the present invention utilizing a combination of the first ionic surfactant and the second ionic surfactant, and heating, a target molecule can be efficiently extracted from the biological sample described above, and a treated sample useful for analysis of the target molecule in the biological sample can be obtained. As the biological sample, a sample containing an individual multicellular organism or a part thereof, or a unicellular organism is preferable, and a sample containing an individual multicellular organism or a part thereof is more preferable.

In a certain embodiment, the biological sample may be a tissue section sample of a multicellular organism (for example, an animal such as a human as described above). The thinner the tissue section is, the more improved a treatment efficiency of the biological sample can be, and therefore a thinner tissue section is more preferable. Therefore, the thickness of the tissue section may be, for example, 0.1 µm to 1 mm, preferably 0.25 to 500 µm, more preferably 0.5 to 120 µm, and still more preferably 4 to 20 µm.

In another certain embodiment, the biological sample may be a fixed tissue sample. The fixed tissue sample is a tissue sample fixed with a component having a fixing action. Examples of such a component include an organic solvent such as an acid, an alcohol, an aldehyde, or a ketone. More specifically, as such a component, formalin (formaldehyde) or glutaraldehyde is preferable, and formalin is more preferable. By heating, release of fixation (for example, cleavage of methylene crosslinking with formalin) in the fixed tissue sample can be promoted, and therefore the treatment of the biological sample can be promoted.

In still another certain embodiment, the biological sample may be a paraffin embedded tissue sample. By heating, the paraffin embedded tissue sample can be suitably treated. That is, by heating, the paraffin can be dissolved and transferred into a solution. Thereafter, the paraffin can be removed from the solution when the temperature of the solution falls below a paraffin dissolution temperature (about 58 to 60°C, although the temperature varies depending on a factor such as the type of paraffin used). For example, when the solution is contained in a container (for example, a tube or a plate), the paraffin can be efficiently solidified on a surface of the container (for example, a wall surface of a tube or a plate). Therefore, according to the method of the present invention, the paraffin can be removed from the liquid phase by transferring the paraffin from the liquid phase to the surface of the container. Therefore, no replacement of the container is required in removal of the paraffin from the solution. The paraffin may decrease an extraction efficiency of the target molecule, and may affect analysis of the target molecule (for example, a decrease in measurement accuracy). Therefore, according to the method of the present invention, even when the biological sample is a paraffin embedded tissue sample, the target molecule can be efficiently extracted from the biological sample without requiring replacement of the container, and the target molecule in the biological sample can be easily analyzed by any analytical method.

In still another certain embodiment, the biological sample may be a formalin fixed paraffin embedded (FFPE) tissue section sample. Details of the formalin fixation, the paraffin embedding, and the tissue section are as described above. Even when the biological sample is an FFPE tissue sample, according to the present invention utilizing the first and second ionic surfactants and heating, treatment of an FFPE tissue section and analysis of a target molecule in the FFPE tissue section by any analytical method can be facilitated.

The solution used in the above (1) contains the first ionic surfactant in addition to the biological sample containing the target molecule.

The first ionic surfactant is an ionic surfactant containing one or both of an anionic moiety and a cationic moiety. The ionic surfactant contains a hydrophilic group containing an ionic moiety and a hydrophobic group as a moiety exhibiting a surfactant action. The hydrophobic group of the moiety exhibiting a surfactant action is typically a linear or branched (preferably linear) carbon chain (preferably an alkyl chain) which may have a cyclic structural moiety (for example, aryl, heteroaryl, cycloalkyl, or non-aromatic heterocycle). The number of carbon atoms constituting such a carbon chain may be, for example, an integer of 6 or more, preferably 8 or more, more preferably 9 or more, still more preferably 10 or more. The number of carbon atoms may be an integer of 30 or less, preferably an integer of 20 or less, more preferably an integer of 18 or less, and still more preferably an integer of 16 or less from a viewpoint of availability, cost, or the like. More specifically, the number of carbon atoms may be, for example, an integer of 6 to 30, preferably 8 to 20, more preferably 9 to 18, still more preferably 10 to 16.

The first ionic surfactant may be in a free form or in a form of a salt. Examples of the salt include a metal salt (for example, a monovalent metal salt such as a sodium salt or a potassium salt, or a divalent metal salt such as a calcium salt or a magnesium salt), an inorganic salt (for example, a halide salt such as a fluoride, a chloride, a bromide, or an iodide, or an ammonium salt), an organic salt (for example, an ammonium salt having an alkyl group as a substituent), and an acid addition salt (for example, a salt with an inorganic acid such as sulfuric acid, hydrochloric acid, hydrobromic acid, nitric acid, or phosphoric acid, or a salt with an organic acid such as acetic acid, oxalic acid, lactic acid, citric acid, trifluoromethanesulfonic acid, or trifluoroacetic acid).

The concentration of the first ionic surfactant is not particularly limited as long as the concentration is sufficient for treating the biological sample, and may be, for example, 0.1 to 20 wt%, preferably 0.2 to 15 wt%, more preferably 0.4 to 12 wt%, still more preferably 0.5 to 10 wt%, and particularly preferably 1 to 8 wt%, although the concentration varies depending on a factor such as the types of the biological sample and the target molecule. When a combination of two or more ionic surfactants (homogeneous or heterogeneous) is used as the first ionic surfactant, each of the ionic surfactants can be used in a concentration as described above. When a combination of two or more ionic surfactants (homogeneous or heterogeneous) is used as the first ionic surfactant, the total concentration of the first ionic surfactant may be, for example, 0.5 to 30 wt%, preferably 0.8 to 25 wt%, more preferably 1 to 20 wt%, still more preferably 1.5 to 15 wt%, and particularly preferably 2 to 10 wt%.

More specifically, as the first ionic surfactant, an anionic surfactant, a cationic surfactant, a zwitterionic surfactant, a combination of an anionic surfactant and a zwitterionic surfactant, or a combination of a cationic surfactant and a zwitterionic surfactant may be used.

The anionic surfactant is a surfactant containing an anionic moiety (for example, a sulfuric acid group, a sulfonic acid group, a carboxylic acid group, or a phosphoric acid group) and not containing a cationic moiety. Examples of the anionic surfactant include a sulfuric acid ester type surfactant, a carboxylic acid type surfactant, a sulfonic acid type surfactant, a phosphoric acid ester type surfactant, and an amino acid type surfactant. Such an anionic surfactant can have a carbon chain having the number of carbon atoms as described above as a hydrophobic group of a moiety exhibiting a surfactant action. The anionic surfactant may be in a free form or in a form of a salt (preferably a metal salt) as described above.

Specific examples of the anionic surfactant include a Cn alkyl sulfuric acid ester, a Cn alkyl ether sulfuric acid ester, a Cn alkyl carboxylic acid, a N-Cn alkanoylsarcosine, a Cn alkyl sulfonic acid, a Cn alkyl benzene sulfonic acid, a Cn alkyl phosphoric acid, and salts thereof. Here, the Cn alkyl represents an alkyl chain (preferably linear) having the number of carbon atoms as described above for the first ionic surfactant. The Cn alkanoyl represents a Cn alkanoyl chain (preferably linear) having the number of carbon atoms as described above for the first ionic surfactant. Therefore, n can correspond to the number of carbon atoms as described above for the first ionic surfactant. Hereinafter, the same applies to a Cn alkyl, a Cn alkanoyl, and n.

Examples of the Cn alkyl sulfuric acid ester or a salt thereof include octyl sulfuric acid, nonyl sulfuric acid, decyl sulfuric acid, undecyl sulfuric acid, dodecyl sulfuric acid, tridecyl sulfuric acid, tetradecyl sulfuric acid, pentadecyl sulfuric acid, hexadecyl sulfuric acid, heptadecyl sulfuric acid, octadecyl sulfuric acid, nonadecyl sulfuric acid, icosyl (eicosyl) sulfuric acid, and salts thereof. As the Cn alkyl sulfuric acid ester or a salt thereof, a salt of a Cn alkyl sulfuric acid ester is preferable, a metal salt of a Cn alkyl sulfuric acid ester is more preferable, and a sodium salt of a Cn alkyl sulfuric acid ester (for example, SDS) is still more preferable.

Examples of the Cn alkyl ether sulfuric acid ester or a salt thereof include polyoxyethylene octyl ether sulfuric acid, polyoxyethylene nonyl ether sulfuric acid, polyoxyethylene decyl ether sulfuric acid, polyoxyethylene undecyl ether sulfuric acid, polyoxyethylene dodecyl ether sulfuric acid, polyoxyethylene tridecyl ether sulfuric acid, polyoxyethylene tetradecyl ether sulfuric acid, polyoxyethylene pentadecyl ether sulfuric acid, polyoxyethylene hexadecyl ether sulfuric acid, polyoxyethylene heptadecyl ether sulfuric acid, polyoxyethylene octadecyl ether sulfuric acid, polyoxyethylene nonadecyl ether sulfuric acid, polyoxyethylene icosyl (eicosyl) ether sulfuric acid, and salts thereof. As the polyoxyethylene Cn alkyl ether sulfuric acid ester or a salt thereof, a salt of the polyoxyethylene Cn alkyl ether sulfuric acid ester is preferable, a metal salt of the polyoxyethylene Cn alkyl ether sulfuric acid ester is more preferable, and a sodium salt of the polyoxyethylene Cn alkyl ether sulfuric acid ester is still more preferable.

Examples of the Cn alkyl carboxylic acid or a salt thereof include octyl carboxylic acid, nonyl carboxylic acid, decyl carboxylic acid, undecyl carboxylic acid, dodecyl carboxylic acid, tridecyl carboxylic acid, tetradecyl carboxylic acid, pentadecyl carboxylic acid, hexadecyl carboxylic acid, heptadecyl carboxylic acid, octadecyl carboxylic acid, nonadecyl carboxylic acid, icosyl (eicosyl) carboxylic acid, and salts thereof. As the Cn alkyl carboxylic acid or a salt thereof, a salt of the Cn alkyl carboxylic acid is preferable, a metal salt of the Cn alkyl carboxylic acid is more preferable, and a sodium salt of the Cn alkyl carboxylic acid is still more preferable.

Examples of the N-Cn alkanoylsarcosine or a salt thereof include N-octanoylsarcosine, N-nonanylsarcosine, N-decanoylsarcosine, N-undecanoylsarcosine, N-dodecanoylsarcosine (N-lauroylsarcosine), N-tridecanoylsarcosine, N-tetradecanoylsarcosine, N-pentadecanoylsarcosine, N-hexadecanoylsarcosine, N-heptadecanoylsarcosine, N-octadecanoylsarcosine, N-nonadecanoylsarcosine, N-icosinoyl (eicosinoyl) sarcosine, and salts thereof. As the N-Cn alkanoylsarcosine or a salt thereof, a salt of the N-Cn alkanoylsarcosine is preferable, a metal salt of the N-Cn alkanoylsarcosine is more preferable, and a sodium salt of the N-Cn alkanoylsarcosine is still more preferable.

Examples of the Cn alkylsulfonic acid or a salt thereof include octylsulfonic acid, nonylsulfonic acid, decylsulfonic acid, undecylsulfonic acid, dodecylsulfonic acid, tridecylsulfonic acid, tetradecylsulfonic acid, pentadecylsulfonic acid, hexadecylsulfonic acid, heptadecylsulfonic acid, octadecylsulfonic acid, nonadecylsulfonic acid, icosyl (eicosyl) sulfonic acid, and salts thereof. As the Cn alkylsulfonic acid or a salt thereof, a salt of a Cn alkylsulfonic acid is preferable, a metal salt of a Cn alkylsulfonic acid is more preferable, and a sodium salt of a Cn alkylsulfonic acid is still more preferable.

Examples of the Cn alkylbenzenesulfonic acid or a salt thereof include octylbenzenesulfonic acid, nonylbenzenesulfonic acid, decylbenzenesulfonic acid, undecylbenzenesulfonic acid, dodecylbenzenesulfonic acid, tridecylbenzenesulfonic acid, tetradecylbenzenesulfonic acid, pentadecylbenzenesulfonic acid, hexadecylbenzenesulfonic acid, heptadecylbenzenesulfonic acid, octadecylbenzenesulfonic acid, nonadecylbenzenesulfonic acid, icosyl (eicosyl) benzenesulfonic acid, and salts thereof. As the Cn alkylbenzenesulfonic acid or a salt thereof, a salt of the Cn alkylbenzenesulfonic acid is preferable, a metal salt of the Cn alkylbenzenesulfonic acid is more preferable, and a sodium salt of the Cn alkylbenzenesulfonic acid is still more preferable.

Examples of the Cn alkyl phosphoric acid or a salt thereof include octyl phosphoric acid, nonyl phosphoric acid, decyl phosphoric acid, undecyl phosphoric acid, dodecyl phosphoric acid, tridecyl phosphoric acid, tetradecyl phosphoric acid, pentadecyl phosphoric acid, hexadecyl phosphoric acid, heptadecyl phosphoric acid, octadecyl phosphoric acid, nonadecyl phosphoric acid, icosyl (eicosyl) phosphoric acid, and salts thereof. As the Cn alkyl phosphoric acid or a salt thereof, a salt of the Cn alkyl phosphoric acid is preferable, a metal salt of the Cn alkyl phosphoric acid is more preferable, and a sodium salt of the Cn alkyl phosphoric acid is still more preferable.

Alternatively, the anionic surfactant may be an anionic surfactant having a steroid skeleton. Typical examples of such an anionic surfactant include a bile acid or a derivative thereof, and salts thereof. More specific examples of such an anionic surfactant include deoxycholic acid, chenodeoxycholic acid, ursodeoxycholic acid, hyodeoxycholic acid, cholic acid, glycocholic acid, hyocholic acid, 5α-cipurinol, lithocholic acid, taurodeoxycholic acid, taurocholic acid, and salts thereof. As such an anionic surfactant, a salt is preferable, a metal salt is more preferable, and a sodium salt is still more preferable.

The cationic surfactant is a surfactant containing a cationic moiety (for example, ammonium or phosphonium) and not containing an anionic moiety. Examples of the cationic surfactant include a quaternary ammonium type surfactant, a quaternary phosphonium type surfactant, and salts thereof. Such a cationic surfactant can have a carbon chain having the number of carbon atoms as described above for the first ionic surfactant as a hydrophobic group of a moiety exhibiting a surfactant action. The cationic surfactant may be in a free form or in a form of a salt (preferably, a salt with a halogen such as fluorine, chlorine, bromine, or iodine) as described above.

Specific examples of the cationic surfactant include a Cn alkyltrimethylammonium halide and a Cn alkyltrimethylphosphonium halide.

Examples of the Cn alkyltrimethylammonium halide include an octyltrimethylammonium halide, a nonyltrimethylammonium halide, a decyltrimethylammonium halide, an undecyltrimethylammonium halide, a dodecyltrimethylammonium halide, a tridecyltrimethylammonium halide, a tetradecyltrimethylammonium halide, a pentadecyltrimethylammonium halide, a hexadecyltrimethylammonium halide, a heptadecyltrimethylammonium halide, an octadecyltrimethylammonium halide, a nonadecyltrimethylammonium halide, and an icosyl (eicosyl) trimethylammonium halide. As the Cn alkyltrimethylammonium halide, a Cn alkyltrimethylammonium chloride (CnTAC) or a Cn alkyltrimethylammonium bromide (CnTAB) is preferable.

Examples of the Cn alkyltrimethylphosphonium halide include an octyltrimethylphosphonium halide, a nonyltrimethylphosphonium halide, a decyltrimethylphosphonium halide, an undecyltrimethylphosphonium halide, a dodecyltrimethylphosphonium halide, a tridecyltrimethylphosphonium halide, a tetradecyltrimethylphosphonium halide, a pentadecyltrimethylphosphonium halide, a hexadecyltrimethylphosphonium halide, a heptadecyltrimethylphosphonium halide, an octadecyltrimethylphosphonium halide, a nonadecyltrimethylphosphonium halide, and an icosyl (eicosyl) trimethylphosphonium halide. As the Cn alkyltrimethylphosphonium halide, a Cn alkyltrimethylphosphonium chloride or a Cn alkyltrimethylphosphonium bromide is preferable.

Alternatively, the cationic surfactant may be a cationic surfactant having a steroid skeleton. Typical examples of such a cationic surfactant include a derivative of a bile acid that has been derivatized so as to have a cationic moiety while losing an anionic moiety, and a salt thereof. As such a cationic surfactant, the salt as described above (preferably, a salt with a halogen such as fluorine, chlorine, bromine, or iodine) is preferable.

The zwitterionic surfactant is a surfactant containing both a cationic moiety (for example, ammonium, phosphonium, or sulfonium) and an anionic moiety (for example: a sulfuric acid group, a sulfonic acid group, a carboxylic acid group, or a phosphoric acid group). For example, a quaternary ammonium-carboxylic acid type surfactant and/or a quaternary ammonium-sulfonic acid type surfactant may be used as the zwitterionic surfactant from a viewpoint of easy availability or the like. Such a zwitterionic surfactant can have a carbon chain having the number of carbon atoms as described above for the first ionic surfactant as a moiety exhibiting a surfactant action. The zwitterionic surfactant may be in a free form or in a form of a salt as described above.

Examples of the quaternary ammonium-carboxylic acid type surfactant include an alkyldimethylaminoacetic acid betaine such as lauryldimethylaminoacetic acid betaine or stearyldimethylaminoacetic acid betaine.

Examples of the quaternary ammonium-sulfonic acid type surfactant include 3-(N,N-dimethyloctylammonio) propanesulfonate (C8APS), 3-(decyldimethylammonio) propanesulfonate (C10APS), 3-(N,N-dimethyldodecylammonio) propanesulfonate (C12APS), 3-(N,N-dimethylmyristylammonio) propanesulfonate (C14APS), 3-(N,N-dimethylpalmitylammonio) propanesulfonate (C16APS), 3-(N,N-dimethylstearylammonio) propanesulfonate (C18APS), dimethylethylammonium propanesulfonate (NDSB-195), 3-[dimethyl-(2-hydroxyethyl) ammonio]-1-propanesulfonate (NDSB-211), 3-(benzenedimethylammonio) propanesulfonate (NDSB-256), CHAPS, CHAPSO, and salts thereof.

Alternatively, the zwitterionic surfactant may be a zwitterionic surfactant having a steroid skeleton. Typical examples of such a zwitterionic surfactant include a derivative of a bile acid that has been derivatized so as to have a cationic moiety, and a salt thereof. More specifically, examples of such a zwitterionic surfactant include CHAPS and CHAPSO.

In a certain embodiment, the first ionic surfactant may be an anionic surfactant, a zwitterionic surfactant, or a combination thereof. By using these surfactants as the first ionic surfactant, the target molecule can be efficiently extracted from the biological sample.

In another certain embodiment, the first ionic surfactant may contain an ionic surfactant having a steroid skeleton. Examples of the ionic surfactant having a steroid skeleton, used in combination with heating of the biological sample include an anionic surfactant having a steroid skeleton as described above and a zwitterionic surfactant having a steroid skeleton as described above. The first ionic surfactant may be preferably a zwitterionic surfactant having a steroid skeleton. By using an ionic surfactant having a steroid skeleton, the target molecule can be efficiently extracted from the biological sample.

In still another certain embodiment, the first ionic surfactant may be a combination of an anionic surfactant and a zwitterionic surfactant. By heating a solution that contains the biological sample containing the target molecule and contains a combination of an anionic surfactant and a zwitterionic surfactant, the target molecule can be efficiently extracted from the biological sample by a synergistic effect of the anionic surfactant and the zwitterionic surfactant.

In still another certain embodiment, the concentration of the zwitterionic surfactant as the first ionic surfactant may be, for example, 0.01 to 10 wt%, preferably 0.05 to 5, and more preferably 0.1 to 1 wt%. With such a concentration, the target molecule can be efficiently extracted from the biological sample. When a combination of an anionic surfactant and a zwitterionic surfactant is used, the concentration of the zwitterionic surfactant may be, for example, 0.01 to 2 wt%, preferably 0.05 to 1 wt%, and more preferably 0.1 to 0.8 wt%, depending on a concentration of the anionic surfactant. With such a concentration, the target molecule can be efficiently extracted from the biological sample, and a sufficient synergistic effect can be exhibited.

The solution used in the above (1) may further contain a solubilizer for an ionic surfactant. Suitable examples of the solubilizer for an ionic surfactant include a nonionic surfactant. According to the present invention, it has been confirmed that presence or absence of the nonionic surfactant has a low negative influence on a signal obtained from an immunological measurement. Examples of the nonionic surfactant include a polyoxyethylene sorbitan fatty acid ester [for example, TWEEN (registered trademark) series (for example: TWEEN 20, TWEEN 40, or TWEEN 80)], polyoxyethylene octylphenyl ether [for example, TRITON (registered trademark) series (for example: Triton X-100, Triton X-114, Triton X-305, Triton X-405, or Triton X-705)], N-D-gluco-N-methylalkanamide [for example, MEGA series (for example: MEGA 8 or MEGA 10)], and a polyoxyethylene alcohol structure-containing nonionic surfactant (for example: an alcohol ethoxylate or a polyoxyethylene-polyoxyalkylene block copolymer). The concentration of the nonionic surfactant is not particularly limited as long as the concentration is a concentration generally used as a solubilizer of a lipid bilayer membrane, and may be, for example, 0.01 to 10 wt%, preferably 0.05 to 5 wt%, more preferably 0.1 to 2 wt%, and preferably 0.2 to 0.7 wt%.

The solution used in the above (1) may further contain another component. Examples of such a component include a chelating agent, a deposition inhibitor, and a reducing agent.

Examples of the chelating agent include ethylenediaminetetraacetic acid (EDTA), glycol ether diamine tetraacetic acid (EGTA), hydroxyethyliminodiacetic acid (HIDA), nitrilotriacetic acid (NTA), hydroxyethylethylenediaminetriacetic acid (HEDTA), ethylenediaminetetra (methylenephosphonic acid) (EDTMP), and salts thereof.

Examples of the reducing agent include dithiothreitol (DTT), dithioerythritol (DTE), 2-mercaptoethylamine (2MEA), 2-mercaptoethanol (2ME), tris-2-carboxyethylphosphine (TCEP), L-cysteine, N-acetyl-L-cysteine, and ascorbic acid.

As the solution used in the above (1), an aqueous solution can be used. Examples of the aqueous solution include water (for example, distilled water, sterile water, sterile distilled water, or pure water) and a buffer, and a buffer is preferable. Examples of the buffer include a phosphate buffer, phosphate buffer saline (PBS), a tartrate buffer, a citrate buffer, an acetate buffer, a glycine buffer, a carbonate buffer, a 2-morpholinoethanesulfonic acid (MES) buffer, a trishydroxymethylaminomethane (Tris) buffer, a borate buffer, a 3-morpholinopropanesulfonic acid (MOPS) buffer, an N,N-bis (2-hydroxyethyl) glycine (Bicine) buffer, a bis(2-hydroxyethyl) iminotris (hydroxymethyl) methane (Bis-Tris) buffer, a 2-[4-(2-hydroxyethyl) 1-piperazinylethanesulfonic acid (HEPES) buffer, and an imidazole buffer. The volume of the solution is not particularly limited because the volume varies depending on a factor such as the size of the biological sample, and may be 10 mL, 100 mL, or a high volume such as more than 1 L. However, when a specific treatment is desired (for example, an efficient treatment of many samples or a rapid treatment), the volume of the solution may be low. Such a low volume may be, for example, 1 µL to 10 mL, preferably 5 µL to 5 mL, and more preferably 10 µL to 1 mL. The pH of the buffer is preferably neutral. More specifically, such a pH may be preferably 5.0 or more, more preferably 5.5 or more, and still more preferably 6.0 or more. The pH may also be preferably 9.0 or less, more preferably 8.5 or less, and still more preferably 8.0 or less. The pH can be measured using a method known in the art. Preferably, as the pH, a value measured at 25°C using a pH meter having a glass electrode can be adopted.

As the solution used in the above (1), a solution in which the biological sample containing the target molecule is mixed with the first ionic surfactant in advance (for example, a solution in which the biological sample containing the target molecule is immersed in a first ionic surfactant-containing liquid in advance), or a solution obtained by mixing the biological sample containing the target molecule with the first ionic surfactant (preparation upon use) can be used. Therefore, the method of the invention may further include mixing the biological sample containing the target molecule with the first ionic surfactant to generate a solution that contains the biological sample containing the target molecule and contains the first ionic surfactant. Such mixing may be performed by adding a solution containing the first ionic surfactant to the biological sample containing the target molecule or by adding the first ionic surfactant to a solution containing the biological sample containing the target molecule and dissolving the first ionic surfactant therein, but may be performed by adding a solution containing the first ionic surfactant to the biological sample containing the target molecule in order to achieve rapid and simple mixing. In addition, mixing can be performed, for example, by stirring using a stirring device or instrument. Mixing time is not particularly limited as long as the biological sample and the first ionic surfactant are sufficiently mixed, and may be, for example, 1 second to 1 minute, preferably 3 seconds to 30 seconds, and more preferably 5 seconds to 15 seconds, although the time varies depending on factors such as the types and the amounts of the biological sample, the target molecule, and the first ionic surfactant. After mixing, the mixed liquid may be left to stand. Standing time is not particularly limited, and may be, for example, a period exceeding one hour, or may be one hour or less (for example, 30 minutes or less, 20 minutes or less, or 10 minutes or less). A mixing temperature is not particularly limited, and can be appropriately set to an appropriate temperature (for example, 4 to 40°C). Alternatively, in view of subsequent heating of the solution in the above (1), the mixing temperature may be a temperature equal to or lower than a heating temperature (for example, lower than 100°C, lower than 95°C, lower than 90°C, lower than 85°C, lower than 80°C, lower than 75°C, lower than 70°C, lower than 65°C, lower than 60°C, lower than 55°C, or lower than 50°C).

In step (1), the solution that contains the biological sample containing the target molecule and contains the first ionic surfactant is heated to a temperature at which denaturation of the biological sample by the first ionic surfactant can be supported. Such a temperature may be, for example, 80°C or higher, preferably 85°C or higher, more preferably 90°C or higher, still more preferably 95°C or higher, and particularly preferably 100°C, although the temperature varies depending on factors such as the types of the biological sample and the target molecule. The heating time may be, for example, 10 seconds to 3 hours, preferably 30 seconds to 2 hours, more preferably 1 minute to 1 hour, and still more preferably 3 minutes to 40 minutes. When subsequent analysis is performed with an automated analyzing device, the heating time may be set according to analysis time, and may be, for example, 5 minutes to 40 minutes, 5 minutes to 30 minutes, or 5 minutes to 20 minutes. Step (1) may be performed by heating the solution that contains the biological sample containing the target molecule and contains the first ionic surfactant while the solution is shaken by a shaker. For example, a heating shaker (thermoshaker) that performs shaking while performing heating can be used.

The method of the present invention may further include a step of cooling the heated solution after step (1). When a paraffin embedded tissue sample such as an FFPE tissue section sample is used as the biological sample, the paraffin can be removed from the solution by cooling as described above. When the method of the present invention further includes a step of cooling the heated solution, a paraffin removing rate can be increased. Therefore, the method of the present invention can be performed rapidly while an advantage of removing the paraffin from the solution is maintained. The heated solution can be cooled, for example, by cooling the heated solution (or the container containing the heated solution) for a certain period of time using a medium controlled to a temperature lower than a paraffin dissolution temperature [for example, contact with cool air, a cooling bath (for example: a water bath or an ice bath), or a cooling unit in a device, or storage in a cooling device such as a refrigerator] or by adding a solution at a temperature lower than the paraffin dissolution temperature (for example, a room temperature solution or a cooled solution) to the heated solution. The solution at a temperature lower than the paraffin dissolution temperature may be a solution containing the second ionic surfactant. The paraffin dissolution temperature is about 58 to 60°C, although the paraffin dissolution temperature varies depending on a factor such as the type of paraffin. Therefore, the temperature lower than the paraffin dissolution temperature may be, for example, lower than 60°C, preferably lower than 58°C, more preferably lower than 50°C, still more preferably lower than 45°C, particularly preferably lower than 40°C, lower than 35°C, lower than 30°C, lower than 25°C, lower than 20°C, lower than 15°C, lower than 10°C, or lower than 5°C. The lower the cooling temperature is, the more rapidly the paraffin can be removed. Note that when the cooling temperature is excessively low, an undesired component may be deposited in the solution depending on factors such as the types and the amounts of the biological sample, the target molecule, and the first ionic surfactant. Therefore, the solution is preferably cooled at a temperature at which such deposition can be avoided. When a medium controlled to a temperature lower than the paraffin dissolution temperature is used, cooling time is not particularly limited, and may be, for example, 1 second to 1 hour, preferably 3 seconds to 30 minutes, more preferably 5 seconds to 10 minutes, still more preferably 10 seconds to 5 minutes, and particularly preferably 15 seconds to 1 minute, although the cooling time varies depending on factors such as the volume of the solution, and the types and the amounts of the biological sample and the target molecule.

### (2) Mixing

Next, the solution obtained in the step (1) is mixed with a second ionic surfactant.

The second ionic surfactant contains an ionic moiety having a charge opposite to that of an ionic moiety in the first ionic surfactant. By using such a second ionic surfactant, a treated sample useful for analysis of the target molecule can be obtained. A reason why the target molecule can be efficiently analyzed by mixing with the second ionic surfactant is unknown, but one reason therefor is presumed to be that the first ionic surfactant contained in the solution containing the first ionic surfactant obtained in the step (1) can be neutralized in terms of charge. Although a mechanism thereof is not clear, it is considered that formation of a mixed micelle of the first ionic surfactant and the second ionic surfactant can reduce the amount of a surfactant monomer in the solution, can reduce a frequency of contact between the surfactant monomer and a target molecule such as a protein or a molecule for a measurement (for example, an antibody used for an immunological measurement), and can reduce a denaturing action of the surfactant monomer on a protein. Furthermore, addition of the second ionic surfactant to the first ionic surfactant further improves the extraction efficiency of the target molecule from the sample, particularly the tissue sample. Therefore, for example, when the target molecule in the biological sample is immunologically measured using an antibody, by mixing the first ionic surfactant and the second ionic surfactant, denaturation of the antibody can be easily suppressed, and the extraction efficiency of the target molecule is further improved. As a result, there is an advantage that the target molecule can be efficiently analyzed (for example, immunologically measured).

The second ionic surfactant is an ionic surfactant containing one or both of an anionic moiety and a cationic moiety. The ionic surfactant contains a hydrophilic group containing an ionic moiety and a hydrophobic group as a moiety exhibiting a surfactant action. The hydrophobic group of the moiety exhibiting a surfactant action is typically a linear or branched (preferably linear) carbon chain (preferably an alkyl chain) which may have a cyclic structural moiety (for example, aryl, heteroaryl, cycloalkyl, or non-aromatic heterocycle). The number of carbon atoms constituting such a carbon chain may be, for example, an integer of 6 or more, preferably 8 or more, more preferably 9 or more, still more preferably 10 or more. The number of carbon atoms may be an integer of 30 or less, preferably an integer of 20 or less, more preferably an integer of 18 or less, and still more preferably an integer of 16 or less from a viewpoint of availability, cost, or the like. More specifically, the number of carbon atoms may be, for example, an integer of 6 to 30, preferably 8 to 20, more preferably 9 to 18, still more preferably 10 to 16.

The second ionic surfactant may be in a free form or in a form of a salt. Examples of the salt include a salt as described above for the first ionic surfactant.

The concentration of the second ionic surfactant is not particularly limited as long as the concentration is sufficient for treating the biological sample, and can be set such that the target molecule can be efficiently extracted from the biological sample and a treated sample useful for analysis of the target molecule can be obtained, although the concentration varies depending on factors such as the type and the concentration of the first ionic surfactant, and the types of the biological sample and the target molecule. The concentration of such a second ionic surfactant may be, for example, 0.2 to 30 wt%, preferably 0.4 to 25 wt%, more preferably 1 to 20 wt%, still more preferably 1.5 to 15 wt%, and particularly preferably 2 to 10 wt% as a concentration after mixing with the solution obtained in the step (1). When a combination of two or more ionic surfactants (homogeneous or heterogeneous) is used as the second ionic surfactant, each of the ionic surfactants can be used in a concentration as described above. When a combination of two or more ionic surfactants (homogeneous or heterogeneous) is used as the second ionic surfactant, the total concentration of the second ionic surfactant may be, for example, 0.5 to 30 wt%, preferably 0.8 to 25 wt%, more preferably 1 to 20 wt%, still more preferably 1.5 to 15 wt%, and particularly preferably 2 to 10 wt%.

More specifically, as the second ionic surfactant, a cationic surfactant, an anionic surfactant, a zwitterionic surfactant, a combination of a cationic surfactant and a zwitterionic surfactant, or a combination of an anionic surfactant and a zwitterionic surfactant may be used.

As the cationic surfactant, the anionic surfactant, and the zwitterionic surfactant used as the second ionic surfactant, those similar to the above-described cationic surfactant, anionic surfactant, and zwitterionic surfactant used as the first ionic surfactant can be used.

In a certain embodiment, the second ionic surfactant may be a cationic surfactant, a zwitterionic surfactant, or a combination thereof. By using these surfactants as the second ionic surfactant, there is an advantage that a treatment of the biological sample can be further promoted.

In another certain embodiment, the second ionic surfactant may contain an ionic surfactant having a steroid skeleton. Examples of the ionic surfactant having a steroid skeleton, used in mixing the heated solution and the ionic surfactant having a steroid skeleton include a cationic surfactant having a steroid skeleton and a zwitterionic surfactant having a steroid skeleton as described above. The second ionic surfactant may be preferably a zwitterionic surfactant having a steroid skeleton. By using an ionic surfactant having a steroid skeleton, there is an advantage that a treatment of the biological sample can be further promoted.

In still another certain embodiment, the second ionic surfactant may contain an ionic surfactant having an alkyl group as a hydrophobic group of a moiety exhibiting a surfactant action. Examples of the ionic surfactant having the alkyl group, used in mixing the heated solution and the ionic surfactant having the alkyl group include, among the cationic surfactant and the zwitterionic surfactant described above, a cationic surfactant and a zwitterionic surfactant each having an alkyl group. The second ionic surfactant may be preferably a cationic surfactant having an alkyl group. By using an ionic surfactant having an alkyl group, for example, there is an advantage that a treatment of the biological sample containing a protein as the target substance can be further promoted.

In still another certain embodiment, the second ionic surfactant may be a combination of a cationic surfactant and a zwitterionic surfactant. By using such a combination, a synergistic effect of a cationic surfactant and a zwitterionic surfactant can be exhibited.

In still another certain embodiment, the second ionic surfactant may be a combination of an ionic surfactant having a steroid skeleton and an ionic surfactant having an alkyl group. By using such a combination, a synergistic effect of an ionic surfactant having a steroid skeleton and an ionic surfactant having an alkyl group can be exhibited.

The second ionic surfactant can be defined based on a relationship with the first ionic surfactant. For example, the number of moles (X1) of the zwitterionic surfactant added to the mixed liquid in step (2) can be defined based on a relationship with the number of moles (Y1) of the anionic surfactant in the solution in step (1). Such a molar ratio (X1/Y1) may be a value of 0.3 or more. Such a molar ratio may be a value of 0.8 to 15, preferably 0.9 to 10, more preferably 1 to 6 from a viewpoint of further improving the treatment efficiency.

For example, when the solution in step (1) contains an anionic surfactant and a zwitterionic surfactant, the number of moles (X2) of the zwitterionic surfactant added to the mixed liquid in step (2) can be defined based on a relationship between the number of moles (Y2) of the anionic surfactant and the number of moles (X2') of the zwitterionic surfactant in the solution in step (1). In this case, since the number of moles of the zwitterionic surfactant (the total number of moles of X2 and X2') finally contained in step (2) contributes to formation of a mixed micelle of the anionic surfactant and the zwitterionic surfactant, a molar ratio "(X2 + X2')/Y2" may be a value of 0.8 or more. Such a molar ratio may be a value of 0.8 to 15, preferably 0.9 to 10, more preferably 1 to 6 from a viewpoint of further improving the treatment efficiency.

The total number of moles (X3) of the second ionic surfactant added to the mixed liquid in step (2) may be within a specific range with respect to the total number of moles (Y3) of the first ionic surfactant in the solution in step (1). Such a molar ratio (X3/Y3) may be a value of 0.3 to 20. Such a molar ratio may be a value of 0.8 to 15, preferably 0.9 to 10, more preferably 1 to 5 from a viewpoint of further improving the treatment efficiency. By setting such a molar ratio for the second ionic surfactant, in the solution containing the biological sample and the first ionic surfactant, charge-based neutralization can be promoted, furthermore, the extraction efficiency of the target molecule can be improved, and the target molecule in the biological sample can be efficiently analyzed.

In a certain embodiment, when the target molecule is a desired molecule such as a protein, the following combination examples of the first ionic surfactant and the second ionic surfactant can be used.

### (Example 1)

First ionic surfactant: anionic surfactant
Second ionic surfactant: cationic surfactant

### (Example 2)

First ionic surfactant: zwitterionic surfactant
Second ionic surfactant: cationic surfactant

### (Example 3)

First ionic surfactant: anionic surfactant
Second ionic surfactant: zwitterionic surfactant

### (Example 4)

First ionic surfactant: combination of anionic surfactant and zwitterionic surfactant
Second ionic surfactant: cationic surfactant

### (Example 5)

First ionic surfactant: combination of anionic surfactant and zwitterionic surfactant
Second ionic surfactant: zwitterionic surfactant

### (Example 6)

First ionic surfactant: anionic surfactant
Second ionic surfactant: combination of cationic surfactant and zwitterionic surfactant

### (Example 7)

First ionic surfactant: zwitterionic surfactant
Second ionic surfactant: combination of cationic surfactant and zwitterionic surfactant

### (Example 8)

First ionic surfactant: combination of anionic surfactant and zwitterionic surfactant
Second ionic surfactant: combination of cationic surfactant and zwitterionic surfactant

When the target molecule is a protein, among the above (Example 1) to (Example 8), (Example 2) to (Example 8) using a zwitterionic surfactant are preferable, (Example 4) to (Example 8) using a first ionic surfactant (a combination of an anionic surfactant and a zwitterionic surfactant) or a second ionic surfactant (a combination of a cationic surfactant and a zwitterionic surfactant) are more preferable, and (Example 8) is still more preferable from a viewpoint of further treating the biological sample or the like.

Mixing in step (2) can be performed, for example, by stirring using a stirring device or instrument. Mixing time is not particularly limited as long as the biological sample and the second ionic surfactant are sufficiently mixed, and may be, for example, 1 second to 10 minutes, preferably 2 seconds to 5 minutes, and more preferably 3 seconds to 1 minute, although the time varies depending on factors such as the types and the amounts of the biological sample, the target molecule, the first ionic surfactant, and the second ionic surfactant. After mixing, the mixed liquid may be left to stand. Standing time may be, for example, a period exceeding one hour, or may be one hour or less (for example, 30 minutes or less, 20 minutes or less, or 10 minutes or less). A mixing temperature is not particularly limited, and can be appropriately set to an appropriate temperature. Such a mixing temperature may be, for example, 1 to 60°C, preferably 2 to 50°C, and more preferably 4 to 40°C.

Mixing in step (2) may be performed by adding the second ionic surfactant or a solution containing the second ionic surfactant to the solution obtained in step (1). Preferably, in order to achieve rapid and simple mixing, mixing may be performed by adding a solution containing the second ionic surfactant to the solution obtained in step (1).

When a solution containing the second ionic surfactant is added to the solution obtained in step (1), the solution containing the second ionic surfactant may further contain another component. Examples of the other component include a nonionic surfactant (for example, those described above), a chelating agent (for example, those described above), and an antifoaming agent (for example, antifoam).

As the solution containing the second ionic surfactant, an aqueous solution can be used. Examples of the aqueous solution include water (for example, distilled water, sterile water, sterile distilled water, or pure water) and a buffer, and a buffer is preferable. Examples of the buffer include those described above. The volume of the solution is not particularly limited because the volume varies depending on a factor such as the size of the biological sample, and may be 10 mL, 100 mL, or a high volume such as more than 1 L. However, when a specific treatment is desired (for example, an efficient treatment of many samples or a rapid treatment), the volume of the solution may be low. Such a low volume may be, for example, 1 µL to 10 mL, preferably 5 µL to 5 mL, and more preferably 10 µL to 1 mL. The volume of the solution containing the second ionic surfactant may be in a range of, for example, 1/10 to 10, preferably 1/5 to 5, more preferably 1/4 to 4, still more preferably 1/3 to 3, particularly preferably 1/2 to 2 (typically 1 : 1) with respect to the volume of the liquid itself (excluding the volume of the biological sample) in the solution used in step (1). The pH of the buffer can be set such that the pH of a mixed liquid generated by mixing in step (2) is neutral or near neutral. For example, when the pH of the solution used in step (1) is neutral, the pH of the buffer may be a pH near neutral. Meanwhile, when the pH of the solution used in step (1) is acidic, the pH of the buffer may be an alkaline pH. The pH of the solution containing the second ionic surfactant and a measurement thereof are similar to those of the solution used in step (1).

### 2. Method for analyzing target molecule

The present invention provides a method for analyzing a target molecule, including the following (1) to (4):
(1) heating a solution that contains a biological sample containing a target molecule and contains a first ionic surfactant;
(2) mixing the heated solution with a second ionic surfactant containing an ionic moiety having a charge opposite to that of an ionic moiety in the first ionic surfactant to generate a mixed liquid (treated sample) of the target molecule, the first ionic surfactant and the second ionic surfactant;
(3) separating the target molecule from the first ionic surfactant and the second ionic surfactant in the mixed liquid; and
(4) analyzing the target molecule.

### (1) Heating of solution and (2) mixing

Heating of the solution in step (1) and mixing in step (2) can be performed in a similar manner to steps (1) and (2) in the treatment method of the present invention.

### (3) Separation of target molecule

In step (3), the target molecule is separated from the first ionic surfactant and the second ionic surfactant. The target molecule can be separated by any method. For example, the separation can be performed using an affinity substance for the target molecule. Examples of the affinity substance for the target molecule include an antibody, an aptamer, and a lectin. Examples of the antibody include a full-length antibody, an antigen-bound fragment of an antibody (for example, F(ab')2, Fab', Fab, or Fv), and a single-chain antibody.

In a certain embodiment, the separation of the target molecule may be a B (bound)/F (free) separation. According to the B/F separation, since the target molecule can be separated from the first and second ionic surfactants and other components contained in the mixed liquid, a component that may affect a measurement of the target component can be removed. The B (bound)/F (free) separation can be performed through the following steps:
(3a) capturing the target molecule in the mixed liquid with an affinity substance for the target molecule, immobilized on a solid phase; and
(3b) washing the solid phase that has captured the target molecule with a washing liquid.

Examples of the solid phase include particles (for example, sepharose beads, agarose beads, or magnetic particles), a support (for example, a membrane), and a container (for example, a plate such as plastic plate, a tube, or a microchannel). The affinity substance can be immobilized on the solid phase covalently or non-covalently. Typically, the capture can be performed by bringing a solution containing the target molecule into contact with the solid phase on which the affinity substance for the target molecule is immobilized. A capture temperature and capture time can be appropriately set.

Washing can be performed using an aqueous solution (preferably a buffer). The number of times of washing is not particularly limited, and may be, for example, 1 to 5 times, and preferably 1 to 3 times. A washing temperature and washing time can be appropriately set.

### (4) Analysis of target molecule

In step (4), the target molecule is analyzed. The target molecule can be analyzed by appropriately selecting an analysis method according to the type of the target molecule. For example, when the target molecule is a protein, the protein can be analyzed by, for example, an immunological measurement using an antibody (preferably a monoclonal antibody) against the target protein. Examples of the immunological measurement include a direct competition method, an indirect competition method, and a sandwich method. Examples of such an immunological measurement include chemiluminescence immunoassay (CLIA) [for example, chemiluminescent enzyme immunoassay (CLEIA)], immunoturbidimetry (TIA), enzyme immunoassay (EIA) (for example, direct competitive ELISA, indirect competitive ELISA, or sandwich ELISA), radioimmunoassay (RIA), a latex agglutination reaction method, fluorescence immunoassay (FIA), an immunochromatography method, and western blotting. When a plurality of proteins is measured, a proteome analysis may be performed. Alternatively, a protein may be measured by mass spectrometry. A protein can also be measured depending on the type of protein. For example, when the protein is an enzyme, the protein can be measured using the amount of change in a substrate of the enzyme or a product after an enzyme reaction, or a coupling factor of the enzyme reaction as an index. When the protein is a binding protein such as a ligand or an adapter, the protein can be measured using a binding amount of the binding protein to a binding target as an index. The measurement is preferably performed by an immunological measurement from a viewpoint of simplicity, rapidity, or the like.

When the target molecule is analyzed by an immunological measurement, for example, the separation step of step (3) and the analysis step of step (4) may be performed as a part of the immunological measurement.

For example, when the target molecule is analyzed by an immunological measurement using a solid phase, the mixed liquid generated in step (2) is brought into contact with an affinity substance for the target molecule, immobilized on the solid phase to bind the target molecule to the solid phase. Thereafter, B/F separation is performed, and the solid phase is optionally washed (step (3)). Next, the affinity substance for the target molecule (labeling affinity substance), to which a detectable labeling substance is bound, is bound to the target molecule bound to the solid phase, B/F separation is performed, the solid phase is optionally washed, and the labeling substance bound to the solid phase is detected (step (4)), whereby the target molecule can be analyzed.

In addition, the mixed liquid generated in step (2) is brought into contact with the labeling affinity substance to bind the labeling substance to the target molecule, next, the mixed liquid is brought into contact with the affinity substance for the target molecule, immobilized on the solid phase to bind the target molecule to the solid phase, then B/F separation is performed, the solid phase is optionally washed (step (3)), and the labeling substance bound to the solid phase is detected (step (4)), whereby the target molecule can be analyzed.

Furthermore, the mixed liquid generated in step (2) is brought into contact with the affinity substance for the target molecule, immobilized on the solid phase, and the labeling affinity substance to bind the target molecule to the solid phase and the labeling substance, then B/F separation is performed, the solid phase is optionally washed (step (3)), and the labeling substance bound to the solid phase is detected (step (4)), whereby the target molecule can be analyzed.

In addition, when the target molecule is analyzed by an immunological measurement using a solid phase, an affinity substance (affinity substance for a solid phase) to which a substance capable of being immobilized on a solid phase is bound can be used instead of a solid phase on which an affinity substance is immobilized. In this case, the solution containing the target molecule and the affinity substance for a solid phase are brought into contact with each other and then brought into contact with the solid phase to bind the target molecule to the solid phase, B/F separation is performed, the solid phase is optionally washed (step (3)), and the labeling substance bound to the solid phase may be detected (step (4)). Alternatively, the mixed liquid, the affinity substance for a solid phase, and the solid phase are brought into contact with each other to bind the target molecule to the solid phase, B/F separation is performed, the solid phase is optionally washed (step (3)), and the labeling substance bound to the solid phase may be detected (step (4)). Alternatively, after the solution containing the target molecule is brought into contact with the affinity substance for a solid phase, the solution is brought into contact with the labeling affinity substance to bind the affinity substance for a solid phase and the labeling affinity substance to the target molecule, then the solution is brought into contact with the solid phase to bind the target molecule to the solid phase, B/F separation is performed, the solid phase is optionally washed (step (3)), and the labeling substance bound to the solid phase may be detected (step (4)).

Examples of the labeling substance include an enzyme (for example, peroxidase, alkaline phosphatase, luciferase, or β-galactosidase), an affinity substance (for example, streptavidin or biotin), a fluorescent substance or a protein (for example, fluorescein, fluorescein isothiocyanate, rhodamine, a green fluorescent protein, or a red fluorescent protein), a light-emitting or light-absorbing substance (for example, luciferin, acridinium, or ruthenium), and a radioactive substance (for example, 3H, 14C, 32P, 35S, or 125I). The detection can be performed using a detection method known in the art depending on a labeling substance.

### Examples

The present invention will be described in more detail with reference to the following Examples, but the present invention is not limited to the following Examples. % described in Examples refers to wt% (w/w% or wt%) unless otherwise specified.

Information on an ionic surfactant used in Examples is presented in Tables A to C below.

**Table A. Information on anionic surfactant**

| Abbreviation | Name | Structure |
|---|---|---|
| SDS | Sodium dodecyl sulfate | 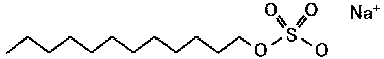 |
| NLS | N-Lauroylsarcosine | 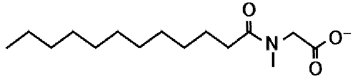 |

**Table B. Information on zwitterionic surfactant**

| Abbreviation | Name | Structure |
|---|---|---|
| CHAPS | 3-[(3-Cholamidopropyl) dimet hylammonio]-1-propanesulfonate | 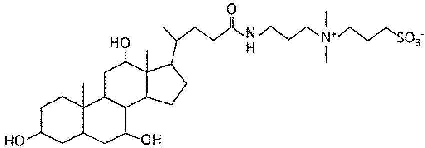 |
| CHAPSO | 3-[(3-Cholamidopropyl) dimet hylammonio] -2-hydroxy-1-propanesulfonate | 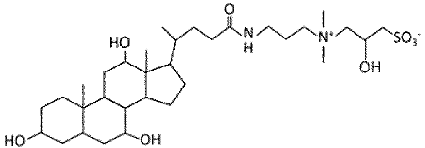 |
| C12APS | N-Dodecyl-N,N-dimethyl-3-ammonio-1-propanesulfonate | 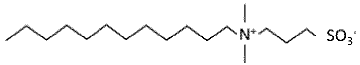 |
| C14APS | 3-(N,N-Dimethylmyristylammonio )propanesulfonate | 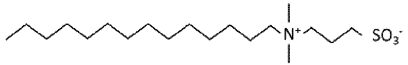 |
| C16APS | 3-(N,N-Dimethylpalmitylammonio )propanesulfonate | 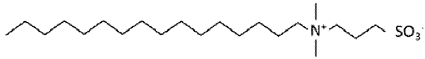 |

**Table C. Information on cationic surfactant**

| Abbreviation | Name | Structure |
|---|---|---|
| C12TAC | Dodecyltrimethylammonium chloride | 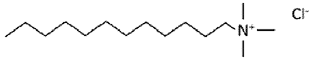 |
| C14TAC | Tetradecyltrimethylammonium chloride | 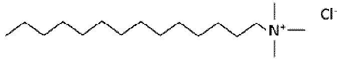 |
| C16TAC | Hexadecyltrimethylammonium chloride | 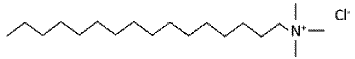 |
| C9TAB | Nonyltrimethylammonium bromide | 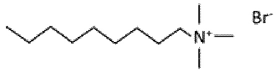 |
| C12TAB | Dodecyltrimethylammonium Bromide | 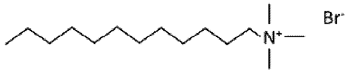 |
| C14TAB | Tetradecyltrimethylammonium bromide | 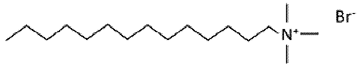 |
| C16TAB | Hexadecyltrimethylammonium bromide | 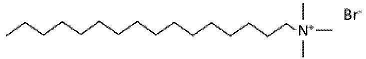 |

### Example 1: Study of carcinoembryonic antigen (CEA) extraction measurement using lung cancer specimen (part 1)

A lung cancer tissue specimen was treated with a first ionic surfactant solution and a second ionic surfactant solution, and CEA was measured by an immunological measurement method using the obtained treated sample.

As a specimen sample, a 10 µm thin section of a formalin fixed paraffin embedded tissue (FFPE tissue, 24 mm × 24 mm block) (lung adenocarcinoma, ID: R14 0624, sample serial no. N858AHN5DQ, Discovery Life Science) of a lung tissue of a patient with lung adenocarcinoma was used. One FFPE section was put into a 1.5 mL assist tube (PP), 200 µL of a first ionic surfactant solution (2 mM disodium ethylenediaminetetraacetate (EDTA·2Na), 100 mM imidazole, pH 7.2) containing a surfactant presented in Table 1 was added thereto, and the mixture was stirred and then heated at 100°C for five minutes. After the heat treatment had been performed for about two minutes, the treated sample was once stirred with a vortex. Thereafter, the sample was cooled with a water bath (temperature: about 20°C) for 30 seconds to solidify the dissolved paraffin on a wall surface of the container, and then 200 µL of a second ionic surfactant solution (50 mM Tris buffer, 2 mM EDTA·2Na, 0.025% Antifoam SI) containing a surfactant presented in Table 1 was added thereto. After stirring, the mixture was allowed to stand at room temperature for 30 minutes to obtain a treated sample. The obtained treated sample was stirred and then subjected to an immunological measurement, and CEA as a target product was measured. For the immunological measurement, Lumipulse (registered trademark) Presto CEA (manufactured by FUJIREBIO Inc.) was used. Specifically, 20 µL of the treated sample and 50 µL of an antibody-bound particle solution (manufactured by FUJIREBIO Inc.) on which an anti-CEA monoclonal antibody was immobilized were dispensed into a reaction vessel, stirred, then incubated at 37°C for eight minutes, and then B (Bond)/F (Free) separation and washing are performed. Into this reaction vessel, 50 µL of an enzyme-labeled antibody solution (manufactured by FUJIREBIO Inc.), which is an anti-CEA monoclonal antibody labeled with an alkaline phosphatase, was further dispensed, stirred, and then incubated at 37°C for eight minutes to perform B/F separation and washing. Thereafter, 200 µL of Lumipulse (registered trademark) substrate solution (manufactured by FUJIREBIO Inc.) containing 3-(2'-spiroadamantane)-4-methoxy-4-(3''-phosphoryloxy) phenyl-1,2-dioxetane disodium salt (AMPPD) as a chemiluminescent substrate was dispensed into the reaction vessel, stirred, then incubated at 37°C for four minutes, and then the amount of luminescence was measured with a luminometer. The actual measurement was performed with a fully automated chemiluminescent enzyme immunoassay system (LUMIPULSE L2400 (manufactured by FUJIREBIO Inc.)).

The obtained count was used as an actual measurement count, and a count obtained by subtracting a background count using a solution containing 2% bovine serum albumin (50 mM Tris buffer, pH 7.2) as a measurement sample was used as a result count.

In addition, CEA was measured similarly using a treated sample obtained by treating an FFPE tissue section using phosphate buffered saline (PBS) instead of the first and second ionic surfactants as a control.

The amount of luminescence (count) obtained by the CEA measurement is presented in Table 1.

**Table 1. Study of CEA extraction measurement using lung cancer specimen (1)**

| Condition | | Control | 1 | 2 | 3 | 4 | 5 |
|---|---|---|---|---|---|---|---|
| First ionic surfactant solution | | | | | | | |
| SDS | anionic | PBS (-) | 2% | 2% | 2% | 2% | 0% |
| NLS | anionic | | 0% | 0% | 0% | 0% | 2% |
| CHAPS | zwitterionic | | 0.6% | 0.6% | 0.6% | 0.6% | 0.6% |
| Tween 20 | nonionic | | 0.43% | 0.43% | 0.43% | 0.43% | 0.43% |

| Second ionic surfactant solution | | | | | | | |
|---|---|---|---|---|---|---|---|
| CHAPS | zwitterionic | PBS (-) | 0% | 5% | 0% | 1.6% | 1.6% |
| C14APS | zwitterionic | | 0% | 0% | 0% | 1.6% | 1.6% |
| C12TAB | cationic | | 0% | 0% | 0% | 0.8% | 0% |
| C9TAB | cationic | | 0% | 0% | 0% | 0% | 0.5% |
| C16TAC | cationic | | 0% | 0% | 5% | 0% | 0% |
| Tween 20 | nonionic | | 0% | 0% | 0% | 2.4% | 2.4% |
| Actual measurement count | | 2399 | 3045 | 43800 | 3633 | 39903 | 35308 |
| | | 2442 | 2949 | 45065 | 3527 | 38893 | 35338 |
| Average of actual measurement count | | 2421 | 2997 | 44433 | 3580 | 39398 | 35323 |
| Result count (background count: 1895) | | 526 | 1102 | 42538 | 1685 | 37503 | 33428 |

As presented in Table 1, by a treatment with a first ionic surfactant solution containing sodium dodecyl sulfate (SDS) and 3-[(3-cholamidopropyl) dimethylammonio] propane sulfonate (CHAPS), a higher count than the control was obtained (condition 1). By a treatment with a second ionic surfactant solution containing CHAPS after the treatment with the first ionic surfactant, the count significantly increased (condition 2). In addition, by a treatment with a second ionic surfactant solution containing hexadecyltrimethylammonium chloride (C16TAC) after the treatment with the first ionic surfactant, the count increased as compared with that in condition 1 in which the second ionic surfactant solution did not contain an ionic surfactant, although an increase level of the count was smaller than that in condition 2 (condition 3). Furthermore, by using a combination of a zwitterionic surfactant (CHAPS and 3-(tetradecyldimethylammonio)propane-1-sulfonate (C14APS)) and a cationic surfactant (dodecyltrimethylammonium bromide (C12TAB) or trimethylnonylammonium bromide (C9TAB)) as a second ionic surfactant, the count significantly increased (conditions 4 and 5).

Even when sodium N-lauroyl sarcosine (NLS) was used as a first ionic surfactant instead of SDS, an increase in counts was observed to the same extent as in the case of SDS (condition 5).

From these results, it was indicated that in order to detect a specific substance from a tissue, treating a tissue specimen with an anionic surfactant and a zwitterionic surfactant, and then treating the tissue specimen with a zwitterionic surfactant or a cationic surfactant were useful in an immunological measurement of an object to be measured contained in an obtained tissue-treated sample.

### Example 2: Study of CEA extraction measurement using lung cancer specimen (part 2)

Using the tissue specimen described in Example 1, types of ionic surfactants contained in a first ionic surfactant solution were studied.

The tissue specimen was treated according to the method described in Example 1 except that a first ionic surfactant solution (2 mM EDTA·2Na, 100 mM imidazole, pH 7.2) containing a surfactant presented in Table 2 and a second ionic surfactant solution (50 mM Tris buffer, 2 mM EDTA·2Na, 0.025% Antifoam SI) containing a surfactant presented in Table 2 were used, and CEA was measured for the obtained treated sample. Results thereof are presented in Table 2.

**Table 2. Study of CEA extraction measurement using lung cancer specimen (2)**

| Condition | | 6 | 7 | 8 | 9 | 10 | 11 |
|---|---|---|---|---|---|---|---|
| First ionic surfactant solution | | | | | | | |
| SDS | anionic | 0% | 2% | 2% | 0% | 2% | 4% |
| CHAPS | zwitterionic | 0% | 0% | 0% | 0.6% | 0.6% | 0.6% |
| Tween 20 | nonionic | 0% | 0% | 0.425% | 0.425% | 0.425% | 0.425% |

| Second ionic surfactant solution | | | | | | | |
|---|---|---|---|---|---|---|---|
| CHAPS | zwitterionic | 1.6% | 1.6% | 1.6% | 1.6% | 1.6% | 1.6% |
| C14APS | zwitterionic | 1.6% | 1.6% | 1.6% | 1.6% | 1.6% | 1.6% |
| C16TAC | cationic | 0.6% | 0.6% | 0.6% | 0.6% | 0.6% | 1.2% |
| C12TAB | cationic | 0.8% | 0.8% | 0.8% | 0.8% | 0.8% | 0.8% |
| Tween 20 | nonionic | 2.4% | 2.4% | 2.4% | 2.4% | 2.4% | 2.4% |
| Actual measurement count | | 6456 | 24383 | 24880 | 13711 | 50858 | 24235 |
| | | 6578 | 24144 | 24980 | 13996 | 51891 | 24581 |
| Average of actual measurement count | | 6517 | 24264 | 24930 | 13854 | 51375 | 24408 |
| Result count (background count: 1808) | | 4709 | 22456 | 23122 | 12046 | 49567 | 22600 |

As presented in Table 2, it was indicated that presence or absence of addition of a nonionic surfactant to a first ionic surfactant solution had almost no influence on a count obtained from the immunological measurement (conditions 7 and 8). Next, as a result of a treatment with a first ionic surfactant solution containing SDS or CHAPS (condition 8 or 9), an increase in count was observed when any surfactant was used, and it was found that the treatment was effective. In particular, the count in the case of using SDS which is an anionic surfactant as a first ionic surfactant was about twice. Furthermore, it was found that when SDS and CHAPS were combined (condition 10), a count largely increased as compared with those in cases where SDS was used alone and CHAPS was used alone, and there was a synergistic effect in an increase in count due to the combination.

When the concentration of SDS in a first ionic surfactant solution was twice and the concentration of C16TAC in a second ionic surfactant solution was twice (condition 11), although a count was sufficiently high, a decrease in count was observed as compared with condition 10. This was considered to be because the concentration of SDS during an immune reaction increased.

### Example 3: Study of CEA extraction measurement using lung cancer specimen (part 3)

Using the tissue specimen described in Example 1, effects of ionic surfactants contained in first and second ionic surfactant solutions were studied.

The tissue specimen was treated according to the method described in Example 1 using a first ionic surfactant solution (2 mM EDTA·2Na, 100 mM imidazole, pH 7.2) containing a surfactant presented in Table 3 and a second ionic surfactant solution (50 mM Tris buffer, 2 mM EDTA·2Na, 0.025% Antifoam SI) containing a surfactant presented in Table 3, and CEA was measured for the obtained treated sample.

**Table 3. Study of CEA extraction measurement using lung cancer specimen (3)**

| Condition 12 | | |
|---|---|---|
| First ionic surfactant solution | | |
| SDS | anionic | 2% |
| CHAPS | zwitterionic | 2% |
| Tween 20 | nonionic | 0.425% |

| Second ionic surfactant solution | | |
|---|---|---|
| CHAPS | zwitterionic | 1.6% |
| C14APS | zwitterionic | 1.6% |
| C16TAC | cationic | 0.6% |
| C12TAB | cationic | 0.8% |
| Tween 20 | nonionic | 2.4% |
| Actual measurement count | | 46545 |
| | | 45378 |
| Average of actual measurement count | | 45962 |
| Result count (background count: 1703) | | 44259 |

As presented in Table 3, a tissue specimen was treated using a first ionic surfactant solution containing an anionic surfactant and a zwitterionic surfactant, and then treated using a second ionic surfactant solution containing a zwitterionic surfactant and a cationic surfactant (condition 12), and as a result, a high count was obtained.

From these results, it was found that, in order to extract and detect a specific substance from a tissue specimen, treatment was performed by a method including an anionic surfactant and a zwitterionic surfactant, and then a treatment was performed by a method including a zwitterionic surfactant and a cationic surfactant, whereby a large increase in count was confirmed, and detection efficiency was improved.

In addition, when a first ionic surfactant solution containing 2% SDS and 0.6% CHAPS in Table 2 was used (condition 10) and when a solution containing 2% SDS and 2% CHAPS in Table 3 was used (condition 12), high counts were obtained to the same extent in the measurement results under these conditions. Therefore, it was confirmed that a synergistic effect of a first ionic surfactant due to a combination of an anionic surfactant and a zwitterionic surfactant could be sufficiently obtained as long as the concentration of the zwitterionic surfactant was about 0.6%.

### Example 4: Study of CEA extraction measurement using lung cancer specimen (part 4)

Using the tissue specimen described in Example 1, effects of ionic surfactants contained in a second ionic surfactant solution were further studied.

The tissue specimen was treated according to the method described in Example 1 except that a first ionic surfactant solution (2 mM EDTA·2Na, 100 mM imidazole, pH 7.2) containing a surfactant presented in Table 4 and a second ionic surfactant solution (50 mM Tris buffer, 2 mM EDTA·2Na, 0.025% Antifoam SI) containing a surfactant presented in Table 4 were used, and CEA was measured for the obtained treated sample. Results thereof are presented in Table 4.

**Table 4. Study of CEA extraction measurement using lung cancer specimen (4)**

| Condition | | 13 | 14 | 15 | 16 | 17 | 18 |
|---|---|---|---|---|---|---|---|
| First ionic surfactant solution | | | | | | | |
| SDS | anionic | 2% | 2% | 2% | 2% | 2% | 2% |
| CHAPS | zwitterionic | 2% | 2% | 2% | 2% | 2% | 2% |
| Tween 20 | nonionic | 0.425% | 0.425% | 0.425% | 0.425% | 0.425% | 0.425% |

| Second ionic surfactant solution | | | | | | | |
|---|---|---|---|---|---|---|---|
| CHAPS | zwitterionic | 5% | 3% | 6% | 7% | 0% | 5% |
| C16TAC | cationic | 0.6% | 0% | 0% | 0% | 0.6% | 0% |
| Background count | | 1772 | 1772 | 1772 | 1772 | 1895 | 1895 |
| Actual measurement count | | 55759 | 23224 | 60640 | 54962 | 2711 | 43800 |
| | | 57295 | 23789 | 60338 | 54506 | 2566 | 45065 |
| Average of actual measurement count | | 56527 | 23507 | 60489 | 54734 | 2639 | 44433 |
| Result count | | 54755 | 21735 | 58717 | 52962 | 744 | 42538 |

As presented in Table 4, as a result of varying the concentration of CHAPS contained in a second ionic surfactant solution to 3%, 6%, and 7%, a high count obtained by containing 3% CHAPS (condition 14) increased by increasing the concentration to 6% CHAPS (condition 15), and the count slightly decreased as compared with 6% when the concentration was further increased to 7% CHAPS (condition 16), but the high count was maintained under all the conditions. In addition, when C16TAC was added to a second ionic surfactant solution containing 5% CHAPS, a high count was obtained (condition 13), and a count obtained using a second ionic surfactant solution containing both CHAPS and C16TAC significantly increased as compared with a count using a second ionic surfactant containing CHAPS or C16TAC (conditions 17 and 18).

From this result, it was found that, in the tissue specimen treated with a first ionic surfactant using an anionic surfactant or a zwitterionic surfactant, a synergistic effect could be obtained by combining a zwitterionic surfactant and a cationic surfactant for a second ionic surfactant.

### Example 5: Study of CEA extraction measurement using colon cancer specimen (part 1)

A cancer tissue specimen different from that in Examples 1 to 4 was also studied.

Using a colon cancer tissue specimen (FFPE tissue, 24 mm × 36 mm block) (Colorectal adenocarcinoma, ID: 110055914, sample serial no. F00023069, Discovery Life Science), the concentration of an ionic surfactant contained in a second ionic surfactant solution was studied.

The tissue specimen was treated according to the method described in Example 1 except that a first ionic surfactant solution having the same composition as that used in Example 4 and a second ionic surfactant solution (50 mM Tris buffer, 2 mM EDTA·2Na, 0.025% Antifoam SI) containing CHAPS at a verification concentration set in the vicinity of a concentration calculated from the CHAPS amount ratio (molar ratio) during a second ionic surfactant treatment to the SDS amount during a first ionic surfactant treatment presented in Table 5 were used, and CEA was measured for the obtained treated sample.

An FFPE tissue section was treated using a solution containing no surfactant instead of the first and second ionic surfactants, and CEA was measured similarly from the obtained treated sample (condition 19).

Results thereof are presented in Table 5 and FIG. 1.

**Table 5. Study of CEA extraction measurement using colon cancer specimen (1)**

| Condition | Ratio of SDS in First ionic surfactant solution versus | | Actual measurement count (average) |
|---|---|---|---|
| | CHAPS in mixed liquid of first and second ionic surfactant solutions (mole) | | |
| 19 | SDS is not contained | - | 1711 |
| 20 | 1 | 0.33 | 2504 |
| 21 | 1 | 1.08 | 20408 |
| 22 | 1 | 1.55 | 25065 |
| 23 | 1 | 2.13 | 24509 |
| 24 | 1 | 3.18 | 21055 |
| 25 | 1 | 4.12 | 19294 |
| 26 | 1 | 5.29 | 18295 |
| 27 | 1 | 1.55 | 26396 |
| 28 | 1 | 10.2 | 14390 |
| 29 | 1 | 12.3 | 10576 |

As presented in Table 5, as a result of containing 1 mol or more of CHAPS during a second ionic surfactant treatment of the tissue specimen with respect to 1 mol of SDS during a first ionic surfactant treatment of the tissue specimen (conditions 21 to 29), a much higher count was obtained than the amount of luminescence obtained using a solution containing no surfactant (condition 19), or the amount of luminescence in which a molar ratio of CHAPS to SDS during a second ionic surfactant treatment was 0.4 or less (condition 20). The count reached a maximum value around a molar ratio of CHAPS of 1.5 (conditions 22 and 27), and decreased at a molar ratio of CHAPS of 2 or more, but a high count was observed even under a condition where the molar ratio of CHAPS to SDS was 12 (condition 29).

From this result, it was indicated that a treatment with a first ionic surfactant containing an anionic surfactant and a subsequent treatment with a second ionic surfactant containing a zwitterionic surfactant were useful in an immunological measurement of an object to be measured contained in a tissue-treated sample obtained not only from a lung cancer tissue specimen but also from a colon cancer tissue specimen.

In addition, it was indicated that the number of molecules of a zwitterionic surfactant contained in a second ionic surfactant solution on an anionic surfactant contained in a first ionic surfactant solution had an effect in a range of a molar ratio of 0.3 to 12, and had a sufficient effect in a range of a molar ratio of 1 to 12. In particular, it was indicated that the effect was higher in a range of a molar ratio of 1 to 10 with respect to the anionic surfactant.

### Example 6: Study of CEA extraction measurement using lung cancer specimen (part 5)

Using the tissue specimen described in Example 1, types of ionic surfactants contained in a second ionic surfactant solution were studied.

One 10 µm thin section of an FFPE tissue specimen was put into an assist tube, 400 µL of a first ionic surfactant solution (2 mM EDTA·2Na, 100 mM imidazole, pH 7.2) containing a surfactant presented in Table 6 was added thereto, and the mixture was stirred and then heated at 100°C for ten minutes. After the heat treatment had been performed for about three minutes, the tube was once stirred, and after completion of heating, the sample was cooled with a water bath for 30 seconds. An operation of a second ionic surfactant treatment and subsequent operations were performed using an automatic dispensing mechanism of LUMIPULSE Presto II (manufactured by FUJIREBIO Inc.) in the apparatus. Specifically, a tube containing a sample (extract) obtained by a treatment with a first ionic surfactant was disposed on LUMIPULSE Presto II, 20 µL of the extract and 20 µL of a second ionic surfactant solution (50 mM Tris buffer, 2 mM EDTA·2Na, 0.025% Antifoam SI) containing a surfactant presented in Table 6 were dispensed into a reaction vessel using a pretreatment 2-step mode installed on LUMIPULSE Presto II, and the mixture was stirred and then incubated at 37°C for 6.5 minutes. 20 µL of sample was dispensed from the same one extract for all the second ionic surfactant treatment conditions. Furthermore, 50 µL of anti-CEA antibody-bound particles was dispensed into the reaction vessel and subjected to an immunological measurement, and CEA as a target product was measured. Immunological measurement operations in an antibody-bound particle dispensing step and subsequent steps were performed according to the method described in Example 1.

**Table 6. Study of CEA extraction measurement using lung cancer specimen (5)**

| Condition | | 30 | 31 | 32 | 33 | 34 |
|---|---|---|---|---|---|---|
| First ionic surfactant solution | | | | | | |
| SDS | anionic | 2% | 2% | 2% | 2% | 2% |
| CHAPS | zwitterionic | 2% | 2% | 2% | 2% | 2% |
| Tween 20 | nonionic | 0.43% | 0.43% | 0.43% | 0.43% | 0.43% |

| Second ionic surfactant solution | | | | | | |
|---|---|---|---|---|---|---|
| CHAPS | zwitterionic | 0% | 0% | 0% | 6% | 3% |
| C12APS | zwitterionic | 0% | 0% | 6% | 0% | 0% |
| C14APS | zwitterionic | 0% | 6% | 0% | 0% | 3% |
| C16TAC | cationic | 0% | 0% | 0% | 0% | 1% |
| Actual measurement count | | 6071 | 22984 | 25178 | 160868 | 196178 |
| | | 7069 | 23070 | 25753 | 160077 | 192153 |
| Average of actual measurement | | 6570 | 23027 | 25466 | 160473 | 194166 |

As presented in Table 6, the zwitterionic surfactant contained in the second ionic surfactant solution was not limited to CHAPS (condition 33), and even when C14APS (condition 31) or C12APS (3-(lauryldimethylammonio) propane-1-sulfonate) (condition 32) was used, a sufficient treatment effect was obtained, and a higher count was observed than a count in the case of using a second ionic surfactant solution containing no surfactant (condition 30) under both of these conditions. When a second ionic surfactant solution containing CHAPS, C14APS, and C16TAC was used, the highest count was observed (condition 34).

From these results, it was indicated that the zwitterionic surfactant contained in the second ionic surfactant solution was effective not only in a case where CHAPS having a steroid skeleton was contained but also in a case where a quaternary ammonium structure having a long chain alkyl was contained. In addition, it was indicated that a synergistic effect could be obtained by mixing a zwitterionic surfactant having a steroid skeleton and a zwitterionic surfactant having an alkyl group.

### Example 7: Study of pepsinogen extraction measurement using gastric cancer specimen

A measurement of extracting pepsinogen I and pepsinogen II from a gastric tissue was studied using a gastric cancer tissue specimen (FFPE tissue, one thin sample slide/specimen from a 24 mm × 24 mm or 24 mm × 36 mm block, estimated section thickness 4 µm) (Adenocarcinoma, ID (sample identification number presented in Table 7): Dst 120286B (gastric cancer (1)), Dst 062551B (gastric cancer (2)), and Dst 023070B (gastric cancer (3)), US Biomax Corporation) and a normal gastric tissue specimen (FFPE tissue, one thin sample slide/specimen from a 24 mm × 24 mm or 24 mm × 36 mm block, estimated section thickness 4 µm) (Normal tissue ID: Dst 17N005A (normal stomach (1)), Dst 07N016A (normal stomach (2)), and Dst 08N001A (normal stomach (3)), US Biomax Inc.).

Each of the sample slides was allowed to stand in a paraffin extender heated to 45°C for five minutes or more, shaved together with paraffin using a replacement blade of a razor, and then put into an assist tube, and 200 µL of a first ionic surfactant solution was added thereto. After stirring, the mixture was heated at 100°C for 10 minutes. After the heat treatment had been performed for about three minutes, the tube was once stirred, and after completion of heating, the sample was cooled with a water bath for 30 seconds. The obtained extract (sample obtained by a treatment with a first ionic surfactant) was disposed on LUMIPULSE Presto II. By the same method as in Example 6, a second ionic surfactant treatment of the extract and an immunological measurement were performed in the LUMIPULSE apparatus, and the dispensed amounts of the extract and the second ionic surfactant solution were each 10 µL. For the immunological measurement, LUMIPULSE Presto pepsinogen I and LUMIPULSE Presto pepsinogen II (both manufactured by FUJIREBIO Inc.) were used. Surfactants contained in the first ionic surfactant solution (2 mM EDTA·2Na, 100 mM imidazole, pH 7.2) or the second ionic surfactant solution (50 mM Tris buffer, 2 mM EDTA·2Na, 0.025% Antifoam SI), and results are presented in Table 7. Note that one extract (a sample obtained by a treatment with a first ionic surfactant) was prepared from each of the sample slides of the gastric cancers or each of the sample slides of the normal stomachs, and each dispensed liquid dispensed from the one extract was treated with each of the second ionic surfactants to prepare a treated sample. That is, the immunological measurement of pepsinogen I or pepsinogen II for each of the gastric cancers or each of the normal stomachs was performed using each treated sample obtained from the same sample slide.

**Table 7. Study of pepsinogen extraction measurement using gastric cancer specimen**

| Condition | | 35 | 36 | 37 | 38 |
|---|---|---|---|---|---|
| First ionic surfactant solution | | | | | |
| SDS | anionic | 2% | 2% | 2% | 2% |
| CHAPS | zwitterionic | 2% | 2% | 2% | 2% |
| Tween 20 | nonionic | 0.43% | 0.43% | 0.43% | 0.43% |

| Second ionic surfactant solution | | | | | |
|---|---|---|---|---|---|
| CHAPS | zwitterionic | 0% | 6% | 3% | 3% |
| C14APS | zwitterionic | 0% | 0% | 3% | 3% |
| C16TAC | cationic | 0% | 0% | 1% | 1% |

| Measurement object | | I pepsinogen I | | | I pepsinogen II |
|---|---|---|---|---|---|
| Average of actual measurement count (n=2) | gastric cancer (1) | 835 | 1425 | 1553 | 1408 |
| | gastric cancer (2) | 849 | 927 | 994 | 3348 |
| | gastric cancer (3) | 904 | 922 | 990 | |
| | normal stomach (1) | 932 | 3269709 | 3888301 | 834460 |
| | normal stomach (2) | 892 | 1158732 | 1939132 | 1013182 |
| | normal stomach (3) | 885 | 575113 | 997557 | 466060 |

As presented in Table 7, in an immunological measurement of a treated sample obtained by a tissue extraction treatment using a first ionic surfactant treatment with an anionic surfactant and a zwitterionic surfactant and a second ionic surfactant treatment with a zwitterionic surfactant or a zwitterionic surfactant and a cationic surfactant, high counts were observed for both pepsinogen I and pepsinogen II in the normal stomachs (conditions 36, 37, and 38: normal stomachs (1), (2), and (3)). Meanwhile, in an immunological measurement in which a solution containing no surfactant was used instead of the second ionic surfactant solution, the count largely decreased (condition 35: normal stomachs (1), (2), and (3)). From this result, it was indicated that the treatment with the second ionic surfactant following the treatment with the first ionic surfactant containing an anionic surfactant and a zwitterionic surfactant was useful for the immunological measurement of the obtained treated sample.

In addition, in the gastric cancer specimens, both the counts of pepsinogen I and pepsinogen II were significantly lower than those in the normal stomach specimens (conditions 36 to 38: gastric cancers (1), (2), and (3)). In general, it is known that expression of pepsinogen is reduced in a gastric cancer tissue, and a clinical condition and the expression of pepsinogen were consistent with each other.

### Reference Example 1: Preparation of predetermined solution

### (1) Preparation of antibody-bound particle liquid for measurement of hepatitis B virus surface antigen (HBsAg)

An anti-HBsAg antibody was immobilized on magnetic beads according to a normal method, and added to a 100 mM Tris buffer (pH 7.2) containing NaCl and BSA to prepare an antibody-bound particle liquid.

### (2) Preparation of enzyme-labeled antibody liquid for HBsAg measurement

Alkaline phosphatase was labeled on an anti-HBsAg antibody according to a normal method, and added to a 50 mM MES buffer (pH 6.8) containing NaCl and BSA to prepare an enzyme-labeled antibody liquid.

### Example 8: Study of HBs antigen extraction measurement using hepatitis B virus-positive hepatocellular carcinoma specimen

Using a hepatitis B virus-positive hepatocellular carcinoma tissue specimen (FFPE tissue, thin sample from a 24 mm × 36 mm block, estimated section thickness 4 µm × 1 section) (Hepatitis B Hepatocellular Carcinoma, ID (specimen identification number presented in Table 8): 763068PB (HBV + hepatic cancer (1)) and 766288PB (HBV + hepatic cancer (2)), BioIVT), a normal liver tissue specimen (FFPE tissue, thin sample from a 24 mm × 24 mm block, estimated section thickness 10 µm × 1 section) (Normal tissue ID: C00033133.7A (normal liver (1)) and C00030252.3A (normal liver (2)), Discovery Life Science), and a non-viral hepatocellular carcinoma tissue specimen (FFPE tissue, thin sample from a 24 mm × 24 mm block, estimated section thickness 5 µm × 2 sections) (Hepatocellular Carcinoma ID: C00027404.3Ee (non-viral liver cancer (1)) and C00080800.1b (non-viral liver cancer (2)), Discovery Life Science), a measurement of extracting an HBs antigen from a liver tissue was studied.

For each of the hepatitis B virus-positive hepatocellular carcinoma tissue specimen and the normal liver tissue specimen, one FFPE tissue section was put into a 1.5 mL assist tube (PP), and a pretreatment liquid was added thereto (Hepatitis B virus-positive hepatocellular carcinoma tissue specimen: 500 µL, normal liver tissue specimen: 300 µL). For the non-viral hepatocellular carcinoma tissue specimen, the sample slide was allowed to stand in a paraffin extender heated to 45°C for five minutes or more, shaved together with paraffin using a replacement blade of a razor, and then put into an assist tube, and a pretreatment liquid was added thereto (300 µL). The tissue sample containing the pretreatment liquid was heated at 100°C under a stirring condition of 1000 rpm for ten minutes, and after completion of heating, the sample was cooled with a water bath for five minutes. The obtained tissue extract (sample obtained by a treatment with a first ionic surfactant) was centrifuged for 30 seconds or more with a small desktop centrifuge, and then disposed on the measuring apparatus (LUMIPULSE). By the same method as in Example 6, the second ionic surfactant treatment of the tissue extract and the immunological measurement were performed in the LUMIPULSE apparatus, and the dispensed amounts of the tissue extract and the second ionic surfactant solution were each 50 µL. The immunological measurement was performed using the antibody-bound particle liquid for HBsAg measurement and the enzyme-labeled antibody liquid for HBsAg measurement described in Reference Example 1. Note that a commercially available HBsAg detection kit may be used as a reagent for detecting HBsAg. Surfactants contained in the first ionic surfactant (2 mM EDTA·2Na, 100 mM imidazole, pH 7.2) and the second ionic surfactant solution (50 mM Tris buffer, 2 mM EDTA·2Na, 0.025% Antifoam SI), and results are presented in Table 8.

**Table 8. Study of HBs antigen extraction measurement using hepatitis B virus-positive hepatocellular carcinoma specimen**

| Condition | | 39 | | 40 | | 41 | |
|---|---|---|---|---|---|---|---|
| First ionic surfactant solution | | | | | | | |
| SDS | anionic | 2% | | 2% | | 2% | |
| CHAPS | zwitterionic | 0.6% | | 0.6% | | 0.6% | |
| Tween 20 | nonionic | 0.43% | | 0.43% | | 0.43% | |

| Second ionic surfactant solution | | | | | | | |
|---|---|---|---|---|---|---|---|
| CHAPS | zwitterionic | 3% | | 3% | | 3% | |
| C14APS | zwitterionic | 3% | | 3% | | 3% | |
| C16TAC | cationic | 1% | | 1% | | 1% | |

| Measurement object | | hepatitis B virus-positive hepatocellular carcinoma | | normal liver | | non-viral hepatocellular carcinoma | |
|---|---|---|---|---|---|---|---|
| Average of actual measurement count (n=2) | | background count | 6370 | background count | 5639 | background count | 6370 |
| | | HBV +hepatic cancer (1) | 1235764 | normal liver (1) | 5170 | non-viral liver cancer (1) | 6028 |
| | | HBV + hepatic cancer (2) | 13513640 | normal liver (2) | 5272 | non-viral liver cancer (2) | 6337 |

As presented in Table 8, in an immunological measurement of a treated sample obtained by a tissue extraction treatment using a first ionic surfactant treatment with an anionic surfactant and a zwitterionic surfactant and a second ionic surfactant treatment with a zwitterionic surfactant and a cationic surfactant, a sample extracted from a hepatitis B virus-positive hepatocellular carcinoma tissue specimen was measured, and as a result, a high count considered to be HBs antigen-positive at a count level was observed (condition 39). Meanwhile, as a result of measuring samples extracted from a normal liver tissue specimen and a non-viral hepatocellular carcinoma tissue specimen, count levels thereof were substantially equal to a background signal obtained using each of the first ionic surfactant solutions as a measurement sample (conditions 40 and 41).

From this result, it was indicated that the tissue extraction treatment using a first ionic surfactant treatment with an anionic surfactant and a zwitterionic surfactant and a second ionic surfactant treatment with a zwitterionic surfactant and a cationic surfactant was useful also in an immunological measurement using a virus-associated antigen such as hepatitis B virus-positive hepatocellular carcinoma as an object to be measured.

## Claims

1. A method for treating a biological sample, the method comprising the following (1) and (2):
(1) heating a solution that contains a biological sample containing a target molecule and contains a first ionic surfactant; and
(2) mixing the heated solution with a second ionic surfactant containing an ionic moiety having a charge opposite to that of an ionic moiety in the first ionic surfactant.

2. The method according to claim 1, wherein the target molecule is a protein.

3. The method according to claim 1 or 2, wherein the biological sample is a tissue section sample, a fixed tissue sample, or a paraffin embedded tissue sample.

4. The method according to any one of claims 1 to 3, wherein the biological sample is a formalin fixed paraffin embedded (FFPE) tissue sample.

5. The method according to any one of claims 1 to 4, wherein the first ionic surfactant is an anionic surfactant, or a zwitterionic surfactant, or a combination thereof.

6. The method according to any one of claims 1 to 5, wherein the first ionic surfactant is an ionic surfactant having an alkyl group as a hydrophobic group, an ionic surfactant having a steroid skeleton, or a combination thereof.

7. The method according to any one of claims 1 to 6, wherein the first ionic surfactant is a combination of an anionic surfactant and a zwitterionic surfactant.

8. The method according to any one of claims 1 to 7, wherein the heating is performed at a temperature of 80°C or higher.

9. The method according to any one of claims 1 to 8, wherein the second ionic surfactant is a cationic surfactant, a zwitterionic surfactant, or a combination thereof.

10. The method according to any one of claims 1 to 9, wherein the second ionic surfactant is an ionic surfactant having a steroid skeleton, an ionic surfactant having an alkyl group as a hydrophobic group, or a combination thereof.

11. The method according to any one of claims 1 to 10, wherein the second ionic surfactant is a combination of a cationic surfactant and a zwitterionic surfactant.

12. The method according to any one of claims 1 to 11, the method comprising the following (1') to (4'):
(1') mixing a biological sample containing a target molecule with a first ionic surfactant to generate a solution that contains the biological sample containing the target molecule and contains the first ionic surfactant;
(2') heating the solution that contains the biological sample containing the target molecule and contains the first ionic surfactant;
(3') cooling the heated solution; and
(4') mixing the cooled solution with the second ionic surfactant.

13. A method for analyzing a target molecule, the method comprising the following (1") to (3"):
(1") treating a biological sample according to the method of any of claims 1-12, thereby generating a mixed liquid of the target molecule, the first ionic surfactant, and the second ionic surfactant;
(2") separating the target molecule from the first ionic surfactant and the second ionic surfactant in the mixed liquid; and
(3") analyzing the target molecule.

14. The method according to claim 13, wherein the separating includes the following (a) and (b):
(a) capturing the target molecule in the mixed liquid with an affinity substance for the target molecule, which is immobilized on a solid phase; and
(b) washing the solid phase that has captured the target molecule with a washing liquid.

15. The method according to claim 13 or 14, wherein the analyzing is performed by an immunological measurement.

## Patentansprüche

1. Verfahren zur Behandlung einer biologischen Probe, wobei das Verfahren die folgenden (1) und (2) umfasst:
(1) das Erhitzen einer Lösung, die eine biologische Probe, enthaltend ein Zielmolekül, und ein erstes ionisches Tensid enthält; und
(2) das Mischen der erhitzten Lösung mit einem zweiten ionischen Tensid, das eine ionische Gruppe mit einer Ladung, die der einer ionischen Gruppe in dem ersten ionischen Tensid entgegengesetzt ist, enthält.

2. Verfahren gemäß Anspruch 1, wobei das Zielmolekül ein Protein ist.

3. Verfahren gemäß Anspruch 1 oder 2, wobei die biologische Probe eine Gewebeschnittprobe, eine fixierte Gewebeprobe oder eine in Paraffin eingebettete Gewebeprobe ist.

4. Verfahren gemäß einem der Ansprüche 1 bis 3, wobei die biologische Probe eine formalinfixierte, in Paraffin eingebettete (FFPE) Gewebeprobe ist.

5. Verfahren gemäß einem der Ansprüche 1 bis 4, wobei das erste ionische Tensid ein anionisches Tensid oder ein zwitterionisches Tensid oder eine Kombination davon ist.

6. Verfahren gemäß einem der Ansprüche 1 bis 5, wobei das erste ionische Tensid ein ionisches Tensid mit einer Alkylgruppe als hydrophobe Gruppe, ein ionisches Tensid mit einem Steroidgerüst oder eine Kombination davon ist.

7. Verfahren gemäß einem der Ansprüche 1 bis 6, wobei das erste ionische Tensid eine Kombination von einem anionischen Tensid und einem zwitterionischen Tensid ist.

8. Verfahren gemäß einem der Ansprüche 1 bis 7, wobei das Erhitzen bei einer Temperatur von 80°C oder höher durchgeführt wird.

9. Verfahren gemäß einem der Ansprüche 1 bis 8, wobei das zweite ionische Tensid ein kationisches Tensid, ein zwitterionisches Tensid oder eine Kombination davon ist.

10. Verfahren gemäß einem der Ansprüche 1 bis 9, wobei das zweite ionische Tensid ein ionisches Tensid mit einem Steroidgerüst, ein ionisches Tensid mit einer Alkylgruppe als hydrophobe Gruppe oder eine Kombination davon ist.

11. Verfahren gemäß einem der Ansprüche 1 bis 10, wobei das zweite ionische Tensid eine Kombination von einem kationischen Tensid und einem zwitterionischen Tensid ist.

12. Verfahren gemäß einem der Ansprüche 1 bis 11, wobei das Verfahren die folgenden (1') bis (4') umfasst:
(1') das Mischen einer biologischen Probe, enthaltend ein Zielmolekül, mit einem ersten ionischen Tensid, um eine Lösung zu erzeugen, die die biologische Probe, enthaltend das Zielmolekül, und das erste ionische Tensid enthält;
(2') das Erhitzen der Lösung, die die biologische Probe, enthaltend das Zielmolekül, und das erste ionische Tensid enthält;
(3') das Abkühlen der erhitzten Lösung; und
(4') das Mischen der abgekühlten Lösung mit dem zweiten ionischen Tensid.

13. Verfahren zur Analyse eines Zielmoleküls, wobei das Verfahren die folgenden (1") bis (3") umfasst:
(1") das Behandeln einer biologischen Probe gemäß dem Verfahren eines der Ansprüche 1-12, wodurch eine gemischte Flüssigkeit des Zielmoleküls, des ersten ionischen Tensids und des zweiten ionischen Tensids erzeugt wird;
(2") das Abtrennen des Zielmoleküls von dem ersten ionischen Tensid und dem zweiten ionischen Tensid in der gemischten Flüssigkeit; und
(3") das Analysieren des Zielmoleküls.

14. Verfahren gemäß Anspruch 13, wobei das Abtrennen die folgenden (a) und (b) umfasst:
(a) das Einfangen des Zielmoleküls in der gemischten Flüssigkeit mit einer Affinitätssubstanz für das Zielmolekül, die auf einer festen Phase immobilisiert ist; und
(b) das Waschen der festen Phase, die das Zielmolekül eingefangen hat, mit einer Waschflüssigkeit.

15. Verfahren gemäß Anspruch 13 oder 14, wobei die Analyse mittels einer immunologischen Messung durchgeführt wird.

## Revendications

1. Procédé de traitement d'un échantillon biologique, le procédé comprenant les points (1) et (2) suivants :
(1) le chauffage d'une solution qui contient un échantillon biologique contenant une molécule cible et contient un premier tensioactif ionique ; et
(2) le mélange de la solution chauffée avec un deuxième tensioactif ionique contenant un fragment ionique présentant une charge opposée à celle d'un fragment ionique dans le premier tensioactif ionique.

2. Procédé selon la revendication 1, dans lequel la molécule cible est une protéine.

3. Procédé selon la revendication 1 ou la revendication 2, dans lequel l'échantillon biologique est un échantillon de coupe de tissu, un échantillon de tissu fixé, ou un échantillon de tissu enrobé de paraffine.

4. Procédé selon l'une quelconque des revendications 1 à 3, dans lequel l'échantillon biologique est un échantillon de tissu fixé au formol et enrobé de paraffine (FFPE).

5. Procédé selon l'une quelconque des revendications 1 à 4, dans lequel le premier tensioactif ionique est un tensioactif anionique, ou un tensioactif zwitterionique, ou une combinaison de ceux-ci.

6. Procédé selon l'une quelconque des revendications 1 à 5, dans lequel le premier tensioactif ionique est un tensioactif ionique présentant un groupe alkyle en tant que groupe hydrophobe, un tensioactif ionique présentant un squelette stéroïdien, ou une combinaison de ceux-ci.

7. Procédé selon l'une quelconque des revendications 1 à 6, dans lequel le premier tensioactif ionique est une combinaison d'un tensioactif anionique et d'un tensioactif zwitterionique.

8. Procédé selon l'une quelconque des revendications 1 à 7, dans lequel le chauffage est effectué à une température de 80 °C ou supérieure.

9. Procédé selon l'une quelconque des revendications 1 à 8, dans lequel le deuxième tensioactif ionique est un tensioactif cationique, un tensioactif zwitterionique, ou une combinaison de ceux-ci.

10. Procédé selon l'une quelconque des revendications 1 à 9, dans lequel le deuxième tensioactif ionique est un tensioactif ionique présentant un squelette stéroïdien, un tensioactif ionique présentant un groupe alkyle en tant que groupe hydrophobe, ou une combinaison de ceux-ci.

11. Procédé selon l'une quelconque des revendications 1 à 10, dans lequel le deuxième tensioactif ionique est une combinaison d'un tensioactif cationique et d'un tensioactif zwitterionique.

12. Procédé selon l'une quelconque des revendications 1 à 11, le procédé comprenant les points (1') à (4') suivants :
(1') le mélange d'un échantillon biologique contenant une molécule cible avec un premier tensioactif ionique pour générer une solution qui contient l'échantillon biologique contenant la molécule cible et contient le premier tensioactif ionique ;
(2') le chauffage de la solution qui contient l'échantillon biologique contenant la molécule cible et contient le premier tensioactif ionique ;
(3') le refroidissement de la solution chauffée ; et
(4') le mélange de la solution refroidie avec le deuxième tensioactif ionique.

13. Procédé d'analyse d'une molécule cible, le procédé comprenant les points (1") à (3") suivants :
(1") le traitement d'un échantillon biologique selon le procédé de l'une quelconque des revendications 1 à 12, pour ainsi générer un liquide mélangé de la molécule cible, du premier tensioactif ionique et du deuxième tensioactif ionique ;
(2") la séparation de la molécule cible du premier tensioactif ionique et du deuxième tensioactif ionique dans le liquide mélangé ; et
(3") l'analyse de la molécule cible.

14. Procédé selon la revendication 13, dans lequel la séparation inclut les points (a) et (b) suivants :
(a) la capture de la molécule cible dans le liquide mélangé avec une substance d'affinité pour la molécule cible, qui est immobilisée sur une phase solide ; et
(b) le lavage de la phase solide qui a capturé la molécule cible avec un liquide de lavage.

15. Procédé selon la revendication 13 ou la revendication 14, dans lequel l'analyse est effectuée par une mesure immunologique.
